# EUROPEAN PATENT SPECIFICATION

(11) **EP 1 066 324 B1**
(45) Date of publication and mention of the grant of the patent: **26.08.2009**
(21) Application number: 99914138.5
(22) Date of filing: 25.03.1999
(51) Int. Cl.: C07K 14/705, C12N 15/12, C12N 15/63, C12N 15/70, C12N 15/79

(54) **NOVEL MAMMALIAN G-PROTEIN COUPLED RECEPTORS HAVING EXTRACELLULAR LEUCINE RICH REPEAT REGIONS**
NEUE G-PROTEIN-GEKOPPELTE REZEPTOREN AUS SÄUGETIEREN MIT EXTRAZELLULÄREN LEUCIN-REICHER REGION
NOUVEAUX RECEPTEURS DE MAMMIFERES COUPLES A LA PROTEINE G ET PRESENTANT DES ZONES DE REPETITION RICHES EN LEUCINE

(30) Priority: 26.03.1998 US 79501 P
(43) Date of publication of application: 10.01.2001
(73) Proprietor: THE BOARD OF TRUSTEES OF THE LELAND STANFORD JUNIOR UNIVERSITY, Stanford, CA 94304 (US); Akzo Nobel N.V., 6824 BM Arnhem (NL)
(72) Inventor: HSUEH, Aaron, J., W., Stanford, CA 94305 (US); HSU, Sheau, Yu, Mountain View, CA 94040 (US); LIANG, Shan-Guang, Palo Alto, CA 94306 (US); VAN DER SPEK, Petrus, Johannes, NL-5342 HM Oss (NL)
(74) Representative: Brasnett, Adrian Hugh
(86) International application number: PCT/US1999/006573
(87) International publication number: WO 1999/048921

(56) References cited:
- EP-A- 0 881 289
- WO-A-99/15545
- US-A- 5 614 363
- US-A- 5 614 363
- US-A- 5 858 716
- EL TAYAR N: "Advances in the molecular understanding of gonadotropins-receptors interactions" MOLECULAR AND CELLULAR ENDOCRINOLOGY, AMSTERDAM, NL, vol. 125, no. 1-2, 20 December 1996 (1996-12-20), pages 65-70, XP002222155 ISSN: 0303-7207
- DATABASE EMBL [Online] 4 December 1997 (1997-12-04), "zi13h07.s1 Soares fetal liver spleen 1NFLS S1 Homo sapiens cDNA clone 430717 3' similar to SW:SLIT_DROME P24014 SLIT PROTEIN PRECURSOR. ;." XP002306452 retrieved from EBI accession no. EM_PRO:AA678095 Database accession no. AA678095
- DATABASE EMBL [Online] 21 March 1995 (1995-03-21), "yc54f09.r1 Stratagene liver (#937224) Homo sapiens cDNA clone IMAGE:84521 5', mRNA sequence." XP002306453 retrieved from EBI accession no. EM_PRO:HS95746 Database accession no. T73957
- NOTHACKER H-P ET AL: "MOLECULAR CLONING OF A NOVEL, PUTATIVE G PROTEIN-COUPLED RECEPTOR FROM SEA ANEMONES STRUCTURALLY RELATED TO MEMBERS OF THE FSH, TSH, LH/CG RECEPTOR FAMILY FROM MAMMALS" BIOCHEMICAL AND BIOPHYSICAL RESEARCH COMMUNICATIONS, ACADEMIC PRESS INC. ORLANDO, FL, US, vol. 197, no. 3, 30 December 1993 (1993-12-30), pages 1062-1069, XP002936032 ISSN: 0006-291X
- HAUSER FRANK ET AL: "Molecular cloning, genomic organization, and developmental regulation of a novel receptor from Drosophila melanogaster structurally related to members of the thyroid-stimulating hormone, follicle-stimulating hormone, luteinizing hormone/choriogonadotropin receptor family from mammals" JOURNAL OF BIOLOGICAL CHEMISTRY, vol. 272, no. 2, 1997, pages 1002-1010, XP002306451 ISSN: 0021-9258
- PERKINS D N ET AL: "XFINGER: A tool for searching and visualising protein fingerprints and patterns" COMPUTER APPLICATIONS IN THE BIOSCIENCES, vol. 12, no. 2, 1996, pages 89-94, XP008039183 ISSN: 0266-7061
- HSU S Y ET AL: "Characterization of 2 LGR genes homologous to gonadotropin and thyrotropin receptors with extracellular leucine-rich repeats (LRR) and a 7-transmembrane region" MOLECULAR BIOLOGY OF THE CELL, vol. 9, no. SUPPL., November 1998 (1998-11), page 215A, XP008039151 & 38TH ANNUAL MEETING OF THE AMERICAN SOCIETY FOR CELL BIOLOGY; SAN FRANCISCO, CALIFORNIA, USA; DECEMBER 12-16, 1998 ISSN: 1059-1524
- HSU SHEAU YU ET AL: "Characterization of two LGR genes homologous to gonadotropin and thyrotropin receptors with extracellular leucine-rich repeats and a G protein-coupled, seven-transmembrane region" MOLECULAR ENDOCRINOLOGY, vol. 12, no. 12, December 1998 (1998-12), pages 1830-1845, XP000857281 ISSN: 0888-8809
- HWANG et al., "Analysis of Expressed Sequence Tags from a Fetal Human Heart cDNA Library", GENOMICS, 1995, Vol. 30, pages 293-298, XP002039974
- HSU et al., "Characterization of Two LGR Genes Homologous to Gonadotropin and Thyrotropin Receptors with Extracellular Leucine-Rich Repeats and a G Protein-Coupled, Seven Transmembrane Region", MOLECULAR ENDOCRINOLOGY, December 1998, Vol. 12, No. 12, pages 1830-1845, XP000857281
- MAZERBOURG ET AL: MOL ENDOCRINOL, vol. 18, no. 9, 2004, pages 2241-2254,

## Description

### INTRODUCTION

### Field of the Invention

The field of this invention is the G-protein coupled receptor family of proteins.

### Background

Gonadotropins (Luteinizing hormone, LH; follicle stimulating hormone, FSH; chorionic gonadotropin, CG) and thyrotropin (TSH)) are essential for the growth and differentiation of gonads and thyroid gland, respectively. These glycoprotein hormones bind specific target cell receptors on the plasma membrane to activate the cAMP-protein kinase A pathway.

The receptors for LH, FSH and TSH belong to the large G-protein-coupled, seven-trans-membrane protein family but are unique in having a large N-terminal extra-cellular (ecto-) domain containing leucine-rich repeats important for interaction with large glycoprotein ligands. Studies suggest that in these receptors, the extra-cellular leucine rich repeat region serves as a "baseball glove" which efficiently catches its corresponding large hormone ligand and optimally orients it for interaction with the seven trans-membrane-helical domain of the receptor.

Because hormones and receptors play a prominent role in a variety of physiological processes, there is continued interest in the identification of novel receptors and their ligands, as well as the genes encoding the same.

### Relevant Literature

References of interest include: el Tayar, N, "Advances in the Molecular Understanding of Gonadotropins-Receptors Interactions," Mol. Cell. Endocrinol. (December 20, 1996).125: 65-70; Bhowmick et al., "Determination of Residues Important in Hormone Binding to the Extracellular Domain of the Luteinizing Hormone/Chorionic Gonadotropin Receptor by Site-Directed Mutagenesis and Modeling," Mol. Endocrinol. (September 1996) 10: 1147-1159; Thomas et al., "Mutational Analyses of the Extracellular Domain of the Full-Length Lutropin/Choriogonadotropin Receptor Suggest Leucine-Rich Repeats 1-6 are Involved in Hormone Binding," Mol. Endocrinol. (June 1996) 10:760-768; Segaloff & Ascoli, "The Gonadotropin Receptors: Insights from the Cloning of their cDNAs," Oxf. Rev. Reprod. Biol. (1992) 14: 141-168; Braun et al., "Amino-Terminal Leucine-Rich Repeats in Gonadotropin Receptors Determine Hormone Selectivity," EMBO J (July 1991) 10: 1885-1890; and Segaloff et al., "Structure of the Lutropin/Choriogonadotropin Receptor," Recent Prog. Horn. Res. (1990) 46: 261-301.
US-A-5 614363 describes a protein having the biological activity of a thyroid stimulating hormone (TSH)
receptor. The following two database entries relating to cDNA sequences are also mentioned in the art:
Database accession no. T73957 (yc54f09.r1 Stratagene liver (#937224) Homo sapiens cDNA clone IMAGE:84521)
Database accession no. AA678095 (zi13h07.s1 Soares fetal liver spleen 1NFLS S1 Homo sapiens cDNA clone 430717).

### SUMMARY OF THE INVENTION

The invention relates to a novel mammalian G-protein coupled receptor having extra-cellular leucine rich repeat domains, i.e. LGR4 (SEQ ID NO:1 and SEQ ID NO:2), and polypeptide compositions thereof, as well as nucleotide compositions encoding the same.

Thus the present invention provides an isolated nucleic acid encoding a mammalian protein, wherein said mammalian protein comprises an amino acid sequence with at least 80% sequence identity to SEQ ID NO: 2.

The invention also provides an isolated nucleic acid, wherein the nucleotide sequence of said nucleic acid comprises a nucleotide sequence having at least 75% sequence identity to the sequence set forth in SEQ ID NO: 1 or the complementary sequence thereof, and an isolated nucleic acid comprising at least 50 contiguous nucleotides of the sequence of SEQ ID NO: 1 or the complementary sequence thereof.

The invention further provides an antibody that binds specifically to a polypeptide comprising the amino acid sequence set forth in SEQ ID NO:2.

In another aspect, the invention provides a non-human transgenic animal, wherein said animal in its native form comprises a gene encoding an amino acid sequence having at least 80% sequence identity to SEQ ID NO:2, and said gene is modified in the transgenic animal by an introduced nucleic acid insertion or deletion in said gene.

The subject proteins, polypeptide and nucleic acid compositions find use in a variety of different applications, including the identification of homologous or related genes; the production of compositions that modulate the expression or function of the subject proteins; in the identification of endogenous ligands for the subject orphan receptors; in the generation of functional binding proteins for the neutralization of the actions of endogenous ligands; in gene therapy; in mapping functional regions of the protein; and in studying associated physiological pathways. In addition, modulation of the gene activity *in vivo* may be useful for prophylactic and therapeutic purposes, and the like.

### BRIEF DESCRIPTION OF THE FIGURES

Fig. 1 provides the nucleotide and amino acid sequence for human LGR4.
Figs. 2 provides a comparison of deduced amino acid sequence of LGR4 and 5 cDNAs and those encoding FSH and LH receptors.

### DESCRIPTION OF THE SPECIFIC EMBODIMENTS

A Novel mammalian G-protein coupled receptor having extra-cellular leucine rich repeat regions (i.e. LGR4) and polypeptide compositions related thereto, as well as nucleic acid compositions encoding the same, are provided. The subject polypeptide and/or nucleic acid compositions find use in a variety of different applications, including the identification of homologous or related genes; for the identification of endogenous ligands for these novel receptors; the production of compositions that modulate the expression or function of the receptors; for gene therapy; for mapping functional regions of the receptors; in studying associated physiological pathways; for *in vivo* prophylactic and therapeutic purposes; as immunogens for producing antibodies; in screening for biologically active agents; and the like.

Before the subject invention is further described, it is to be understood that the invention is not limited to the particular embodiments of the invention described below, as variations of the particular embodiments may be made and still fall within the scope of the appended claims. It is also to be understood that the terminology employed is for the purpose of describing particular embodiments, and is not intended to be limiting. Instead, the scope of the present invention will be established by the appended claims.

In this specification and the appended claims, the singular forms "a," "an," and "the" include plural reference unless the context clearly dictates otherwise. Unless defined otherwise, all technical and scientific terms used herein have the same meaning as commonly understood to one of ordinary skill in the art to which this invention belongs.

### CHARACTERIZATION OF LGR4.

LGR4, is a novel mammalian receptor of the G-protein coupled, seven trans-membrane family of proteins, specifically the subfamily of G-protein coupled seven trans-membrane proteins which are characterized by the presence of extra-cellular leucine rich repeat regions. As such, this protein has trans-membrane segments and extra-cellular regions similar to those found in the known LH, FSH, and TSH receptors. In other words, this protein has both a G-protein coupled seven trans-membrane region and a leucine rich repeat extra-cellular domain. The N-terminal extra-cellular domains of these proteins also show high homology with Drosophila Slit and Toll proteins having leucine rich repeats. This protein is expressed in diverse tissues.

The human LGR4 gene has a nucleotide sequence as shown in SEQ ID NO:01. The human LGR4 gene product has an amino acid sequence as shown in SEQ ID NO:02. LGR4 is expressed in a plurality of different tissue types, including ovary, testis, adrenal, placenta, liver, kidney and intestine.

The human LGR5 gene has a nucleotide sequence as shown in SEQ ID NO:03. The LGR5 gene product has an amino acid sequence as shown in SEQ ID NO:04.

. The human LGR7 short form gene has a nucleotide sequence as shown in SEQ ID NO:05. The LGR7 short form gene product has an amino acid sequence as shown in SEQ ID NO:06. The human LGR7 long form gene has a nucleotide sequence as shown in SEQ ID NO:07. The LGR7 long form gene product has an amino acid sequence as shown in SEQ ID NO:08.

### IDENTIFICATION OF LGR4.

Homologs of *LGR4,* are identified by any of a number of methods. A fragment of the provided cDNA may be used as a hybridization probe against a cDNA library from the target organism of interest, where low stringency conditions are used. The probe may be a large fragment, or one or more short degenerate primers.

Nucleic acids having sequence similarity are detected by hybridization under low stringency conditions, for example, at 50°C and 6×SSC (0.9 M sodium chloride/0.09 M sodium citrate) and remain bound when subjected to washing at 55°C in 1×SSC (0.15 M sodium chlorine/0.015 M sodium citrate). Sequence identity may be determined by hybridization under stringent conditions, for example, at 50°C or higher and 0.1×SSC (15 mM sodium chloride/01.5 mM sodium citrate). Nucleic acids having a region of substantial identity to the provided *LGR4,* sequence, e.g. allelic variants, genetically altered versions of the gene, etc., bind to the provided sequences under stringent hybridization conditions. By using probes, particularly labeled probes of DNA sequences, one can isolate homologous or related genes. The source of homologous genes may be any species, e.g., primate species, particularly human; rodents, such as rats and mice; canines; felines; bovines; ovines; equines; yeast; nematodes; etc.

Between mammalian species, *e.g.,* human and mouse, homologs have substantial sequence similarity, *e.g.* at least 75% sequence identity, usually at least 90%, more usually at least 95% between nucleotide sequences. Sequence similarity is calculated based on a reference sequence, which may be a subset of a larger sequence, such as a conserved motif, coding region, flanking region, *etc.* A reference sequence will usually be at least about 18 nt long, more usually at least about 30 nt long, and may extend to the complete sequence that is being compared. Algorithms for sequence analysis are known in the art, such as BLAST, described in Altschul et al. (1990), J. Mol. Biol. 215:403-10. Unless specified otherwise, all sequence analysis numbers provided herein are as determined with the BLAST program using default settings. The sequences provided herein are essential for recognizing *LGR4, LGR5* and *LGR7*-related and homologous proteins in database searches.

### LGR4 NUCLEIC ACID COMPOSITIONS

Nucleic acids encoding LGR4 may be cDNA or genomic DNA or a fragment thereof. The term "*LGR4* gene, " shall be intended to mean the open reading frame encoding specific LGR4 polypeptide, and *LGR4* introns, as well as adjacent 5' and 3' non-coding nucleotide sequences involved in the regulation of expression, up to about 20 kb beyond the coding region, but possibly further in either direction. The gene may be introduced into an appropriate vector for extra-chromosomal maintenance or for integration into a host genome.

The term "cDNA" as used herein is intended to include all nucleic acids that share the arrangement of sequence elements found in native mature mRNA species, where sequence elements are exons and 3' and 5' non-coding regions. Normally mRNA species have contiguous exons, with the intervening introns, when present, removed by nuclear RNA splicing, to create a continuous open reading frame encoding an LGR4 protein.

A genomic sequence of interest comprises the nucleic acid present between the initiation codon and the stop codon, as defined in the listed sequences, including all of the introns that are normally present in a native chromosome. It may further include the 3' and 5' untranslated regions found in the mature mRNA. It may further include specific transcriptional and translational regulatory sequences, such as promoters, enhancers, *etc.,* including about 1 kb, but possibly more, of flanking genomic DNA at either the 5' or 3' end of the transcribed region. The genomic DNA may be isolated as a fragment of 100 kbp or smaller; and substantially free of flanking chromosomal sequence. The genomic DNA flanking the coding region, either 3' or 5', or internal regulatory sequences as sometimes found in introns, contains sequences required for proper tissue and stage specific expression.

The sequence of the 5' flanking region may be utilized for promoter elements, including enhancer binding sites, that provide for developmental regulation in tissues where *LGR4* is expressed. The tissue specific expression is useful for determining the pattern of expression, and for providing promoters that mimic the native pattern of expression. Naturally occurring polymorphisms in the promoter region are useful for determining natural variations in expression, particularly those that may be associated with disease.

Alternatively, mutations may be introduced into the promoter region to determine the effect of altering expression in experimentally defined systems. Methods for the identification of specific DNA motifs involved in the binding of transcriptional factors are known in the art, *e.g.* sequence similarity to known binding motifs, gel retardation studies, *etc.* For examples, see Blackwell et al. (1995), Mol. Med. 1:194-205; Mortlock et al. (1996), Genome Res. 6:327-33; and Joulin and Richard-Foy (1995), Eur. J. Biochem. 232:620-626.

The regulatory sequences may be used to identify *cis* acting sequences required for transcriptional or translational regulation of *LGR4* expression, especially in different tissues or stages of development, and to identify *cis* acting sequences and *trans*-acting factors that regulate or mediate *LGR4* expression. Such transcription or translational control regions may be operably linked to an *LGR4* gene in order to promote expression of wild type or altered LGR4 or other proteins of interest in cultured cells, or in embryonic, fetal or adult tissues, and for gene therapy.

The nucleic acid compositions of the subject invention may encode all or a part of the subject polypeptides. Double or single stranded fragments may be obtained of the DNA sequence by chemically synthesizing oligonucleotides in accordance with conventional methods, by restriction enzyme digestion, by PCR amplification, *etc*. For the most part, DNA fragments will be of at least 15 nt, usually at least 18 nt or 25 nt, and may be at least about 50 nt. Such small DNA fragments are useful as primers for PCR, hybridization screening probes, *etc*. Larger DNA fragments, *i.e.* greater than 100 nt are useful for production of the encoded polypeptide. For use in amplification reactions, such as PCR, a pair of primers will be used. The exact composition of the primer sequences is not critical to the invention, but for most applications the primers will hybridize to the subject sequence under stringent conditions, as known in the art. It is preferable to choose a pair of primers that will generate an amplification product of at least about 50 nt, preferably at least about 100 nt. Algorithms for the selection of primer sequences are generally known, and are available in commercial software packages. Amplification primers hybridize to complementary strands of DNA, and will prime towards each other.

The *LGR4* gene is isolated and obtained in substantial purity, generally as other than an intact chromosome. Usually, the DNA will be obtained substantially free of other nucleic acid sequences that do not include an *LGR4* sequence or fragment thereof, generally being at least about 50%, usually at least about 90% pure and are typically "recombinant", *i.e*. flanked by one or more nucleotides with which it is not normally associated on a naturally occurring chromosome.

The DNA may also be used to identify expression of the gene in a biological specimen. The manner in which one probes cells for the presence of particular nucleotide sequences, as genomic DNA or RNA, is well established in the literature and does not require elaboration here. DNA or mRNA is isolated from a cell sample. The mRNA may be amplified by RT-PCR, using reverse transcriptase to form a complementary DNA strand, followed by polymerase chain reaction amplification using primers specific for the subject DNA sequences. Alternatively, the mRNA sample is separated by gel electrophoresis, transferred to a suitable support, *e.g*. nitrocellulose, nylon, *etc.,* and then probed with a fragment of the subject DNA as a probe. Other techniques, such as oligonucleotide ligation assays, *in situ* hybridizations, and hybridization to DNA probes arrayed on a solid chip may also find use. Detection of mRNA hybridizing to the subject sequence is indicative of *LGR4* gene expression in the sample.

The sequence of an *LGR4* gene, including flanking promoter regions and coding regions, may be mutated in various ways known in the art to generate targeted changes in promoter strength, sequence of the encoded protein, *etc.* The DNA sequence or protein product of such a mutation will usually be substantially similar to the sequences provided herein, *i.e*. will differ by at least one nucleotide or amino acid, respectively, and may differ by at least two but not more than about ten nucleotides or amino acids. The sequence changes may be substitutions, insertions, deletions, or a combination thereof. Deletions may further include larger changes, such as deletions of a domain or exon. Other modifications of interest include epitope tagging, *e.g*. with the FLAG system, HA, *etc.* For studies of subcellular localization, fusion proteins with green fluorescent proteins (GFP) may be used.

Techniques for *in vitro* mutagenesis of cloned genes are known. Examples of protocols for site specific mutagenesis may be found in Gustin et al. (1993), Biotechniques 14:22; Barany (1985), Gene 37:111-23; Colicelli et al. (1985), Mol. Gen. Genet. 199:537-9; and Prentki et al. (1984), Gene 29:303-13. Methods for site specific mutagenesis can be found in Sambrook et al., Molecular Cloning: A Laboratory Manual, CSH Press 1989, pp. 15.3-15.108; Weiner et al. (1993), Gene 126:35-41; Sayers et al. (1992), Biotechniques 13:592-6; Jones and Winistorfer (1992), Biotechniques 12:528-30; Barton et al. (1990), Nucleic Acids Res 18:7349-55; Marotti and Tomich (1989), Gene Anal. Tech. 6:67-70; and Zhu (1989), Anal Biochem 177:120-4. Such mutated genes may be used to study structure-function relationships of LGR4 or to alter properties of the protein that affect its function or regulation.

### LGR4 POLYPEPTIDES

Also provided by the subject invention are LGR4 polypeptide compositions. The term polyeptide composition as used herein refers to both the full length proteins as well as portions or fragments thereof. Also included in this term are variations of the naturally occurring proteins, where such variations are homologous or substantially similar to the naturally occurring protein, be the naturally occurring protein the human protein, mouse protein, or protein from some other species which naturally expresses an LGR4 protein, usually a mammalian species. A candidate homologous protein is substantially similar to an LGR4 protein of the subject invention, and therefore is an LGR4 protein of the subject invention, if the candidate protein has a sequence that has at least about 80%, usually at least about 90% and more usually at least about 98% sequence identity with an LGR4, protein, as measured by BLAST, supra. In the following description of the subject invention, the term "LGR4 -protein" is used to refer not only to the human LGR4 protein, but also to homologs thereof expressed in non-human species, e.g. murine, rat and other mammalian species.

The subject gene may be employed for producing all or portions of LGR4 polypeptides. By "LGR4 polypeptide/protein" is meant an amino acid sequence encoded by an open reading frame (ORF) of *LGR4* genes, including the full-length native polypeptide and fragments thereof, particularly biologically active fragments and/or fragments corresponding to functional domains, e.g. extra-cellular regions; and including fusions of the subject polypeptides to other proteins or parts thereof, e.g. chimeric proteins. For expression, an expression cassette may be employed. The expression vector will provide a transcriptional and translational initiation region, which may be inducible or constitutive, where the coding region is operably linked under the transcriptional control of the transcriptional initiation region, and a transcriptional and translational termination region. These control regions may be native to an *LGR4* gene, or may be derived from exogenous sources.

Expression vectors generally have convenient restriction sites located near the promoter sequence to provide for the insertion of nucleic acid sequences encoding heterologous proteins. A selectable marker operative in the expression host may be present. Expression vectors may be used for the production of fusion proteins, where the exogenous fusion peptide provides additional functionality, i.e. increased protein synthesis, stability, reactivity with defined antisera, an enzyme marker, e.g. β-galactosidase, etc.

Expression cassettes may be prepared comprising a transcription initiation region, the gene or fragment thereof, and a transcriptional termination region. Of particular interest is the use of sequences that allow for the expression of functional epitopes or domains, usually at least about 8 amino acids in length, more usually at least about 15 amino acids in length, to about 25 amino acids, and up to the complete open reading frame of the gene. After introduction of the DNA, the cells containing the construct may be selected by means of a selectable marker, the cells expanded and then used for expression.

LGR4 polypeptides may be expressed in prokaryotes or eukaryotes in accordance with conventional ways, depending upon the purpose for expression. For large scale production of the protein, a unicellular organism, such as *E*. *coli. B. subtilis, S. cerevisiae,* insect cells in combination with baculovirus vectors, or cells of a higher organism such as vertebrates, particularly mammals, *e.g*. COS 7 cells, may be used as the expression host cells. In some situations, it is desirable to express the *LGR4,* gene in eukaryotic cells, where the LGR4 protein will benefit from native folding and post-translational modifications. Small peptides can also be synthesized in the laboratory. Polypeptides that are subsets of the complete LGR4, sequence may be used to identify and investigate parts of the protein important for function or to raise antibodies directed against these regions.

For production of the extracellular domain of the LGR4 receptor, the anchored receptor approach as described in Osuga et al, Mol. Endocrinol. (1997) 11: 1659-1668 may be employed. Likewise, the chimeric receptor approach described in Kudo et al, J Biol. Chem. (1996) 271; 22470-22478 may be used.

Such peptides find use in the identification of endogenous ligands and in drug screening for agonists and atangonists using methods described in Osuga, supra. Solubilized extracellular domains find use as therapeutic agents, e.g. in the neutralization of the action of endogenous ligands.

With the availability of the protein or fragments thereof in large amounts, by employing an expression host, the protein may be isolated and purified in accordance with conventional ways. A lysate may be prepared of the expression host and the lysate purified using HPLC, exclusion chromatography, gel electrophoresis, affinity chromatography, or other purification technique. The purified protein will generally be at least about 80% pure, preferably at least about 90% pure, and may be up to and including 100% pure. Pure is intended to mean free of other proteins, as well as cellular debris.

The expressed LGR4 polypeptides are useful for the production of antibodies, where short fragments provide for antibodies specific for the particular polypeptide, and larger fragments or the entire protein allow for the production of antibodies over the surface of the polypeptide. Antibodies may be raised to the wild-type or variant forms of LGR4. Antibodies may be raised to isolated peptides corresponding to these domains, or to the native protein.

Antibodies are prepared in accordance with conventional ways, where the expressed polypeptide or protein is used as an immunogen, by itself or conjugated to known immunogenic carriers, *e.g*. KLH, pre-S HBsAg, other viral or eukaryotic proteins, or the like. Various adjuvants may be employed, with a series of injections, as appropriate. Both polyclonal and monoclonal antibodies may be produced. For monoclonal antibodies, after one or more booster injections, the spleen is isolated, the lymphocytes immortalized by cell fusion, and then screened for high affinity antibody binding. The immortalized cells, *i.e*. hybridomas, producing the desired antibodies may then be expanded. For further description, see Monoclonal Antibodies: A Laboratory Manual, Harlow and Lane eds., Cold Spring Harbor Laboratories, Cold Spring Harbor, New York, 1988. If desired, the mRNA encoding the heavy and light chains may be isolated and mutagenized by cloning in *E*. *coli,* and the heavy and light chains mixed to further enhance the affinity of the antibody. Alternatives to *in vivo* immunization as a method of raising antibodies include binding to phage "display" libraries, usually in conjunction with *in vitro* affinity maturation.

### DIAGNOSTIC USES

The subject nucleic acid and/or polypeptide compositions may be used to analyze a patient sample for the presence of polymorphisms associated with a disease state or genetic predisposition to a disease state. Biochemical studies may be performed to determine whether a sequence polymorphism in an *LGR4* coding region or control regions is associated with disease. Disease associated polymorphisms may include deletion or truncation of the gene, mutations that alter expression level, that affect the activity of the protein, and the like.

Changes in the promoter or enhancer sequence that may affect expression levels of *LGR4* can be compared to expression levels of the normal allele by various methods known in the art. Methods for determining promoter or enhancer strength include quantitation of the expressed natural protein; insertion of the variant control element into a vector with a reporter gene such as β-galactosidase, luciferase, chloramphenicol acetyltransferase, *etc.,* that provides for convenient quantitation; and the like.

A number of methods are available for analyzing nucleic acids for the presence of a specific sequence, *e.g*. a disease associated polymorphism. Where large amounts of DNA are available, genomic DNA is used directly. Alternatively, the region of interest is cloned into a suitable vector and grown in sufficient quantity for analysis. Cells that express LGR4, LGR5 or LGR7 may be used as a source of mRNA, which may be assayed directly or reverse transcribed into cDNA for analysis. The nucleic acid may be amplified by conventional techniques, such as the polymerase chain reaction (PCR), to provide sufficient amounts for analysis. The use of the polymerase chain reaction is described in Saiki, et al. (1985), Science 239:487, and a review of techniques may be found in Sambrook, et al. Molecular Cloning: A Laboratory Manual, CSH Press 1989, pp. 14.2-14.33. Alternatively, various methods are known in the art that utilize oligonucleotide ligation as a means of detecting polymorphisms, for examples see Riley et al. (1990), Nucl. Acids Res. 18:2887-2890; and Delahunty et al. (1996), Am. J. Hum. Genet. 58:1239-1246.

A detectable label may be included in an amplification reaction. Suitable labels include fluorochromes, e.g. fluorescein isothiocyanate (FITC), rhodamine, Texas Red, phycoerythrin, allophycocyanin, 6-carboxyfluorescein (6-FAM), 2',7'-dimethoxy-4',5'-dichloro-6-carboxyfluorescein (JOE), 6-carboxy-X-rhodamine (ROX), 6-carboxy-2',4',7',4,7-hexachlorofluorescein (HEX), 5-carboxyfluorescein (5-FAM) or N,N,N',N'-tetramethyl-6-carboxyrhodamine (TAMRA), radioactive labels, *e.g.* ¹²P, ³⁵S, ³H; *etc.* The label may be a two stage system, where the amplified DNA is conjugated to biotin, haptens, *etc*. having a high affinity binding partner, *e.g*. avidin, specific antibodies, *etc*., where the binding partner is conjugated to a detectable label. The label may be conjugated to one or both of the primers. Alternatively, the pool of nucleotides used in the amplification is labeled, so as to incorporate the label into the amplification product.

The sample nucleic acid, *e.g*. amplified or cloned fragment, is analyzed by one of a number of methods known in the art. The nucleic acid may be sequenced by dideoxy or other methods, and the sequence of bases compared to a wild-type *LGR4* sequence. Hybridization with the variant sequence may also be used to determine its presence, by Southern blots, dot blots, *etc.* The hybridization pattern of a control and variant sequence to an array of oligonucleotide probes immobilized on a solid support, as described in US 5,445,934, or in WO 95/35505 (the disclosures of which are herein incorporated by reference), may also be used as a means of detecting the presence of variant sequences. Single strand conformational polymorphism (SSCP) analysis, denaturing gradient gel electrophoresis (DGGE), and heteroduplex analysis in gel matrices are used to detect conformational changes created by DNA sequence variation as alterations in electrophoretic mobility. Alternatively, where a polymorphism creates or destroys a recognition site for a restriction endonuclease, the sample is digested with that endonuclease, and the products size fractionated to determine whether the fragment was digested. Fractionation is performed by gel or capillary electrophoresis, particularly acrylamide or agarose gels.

Screening for mutations in *LGR4* may be based on the functional or antigenic characteristics of the protein. Protein truncation assays are useful in detecting deletions that may affect the biological activity of the protein. Various immunoassays designed to detect polymorphisms in LGR4 proteins may be used in screening. Where many diverse genetic mutations lead to a particular disease phenotype, functional protein assays have proven to be effective screening tools. The activity of the encoded LGR4 protein may be determined by comparison with the wild-type protein.

Antibodies specific for LGR4 proteins may be used in staining or in immunoassays. Samples, as used herein, include biological fluids such as semen, blood, cerebrospinal fluid, tears, saliva, lymph, dialysis fluid and the like; organ or tissue culture derived fluids; and fluids extracted from physiological tissues. Also included in the term are derivatives and fractions of such fluids. The cells may be dissociated, in the case of solid tissues, or tissue sections may be analyzed. Alternatively a lysate of the cells may be prepared.

Diagnosis may be performed by a number of methods to determine the absence or presence or altered amounts of normal or abnormal LGR4 in patient cells. For example, detection may utilize staining of cells or histological sections, performed in accordance with conventional methods. Cells are permeabilized to stain cytoplasmic molecules. The antibodies of interest are added to the cell sample, and incubated for a period of time sufficient to allow binding to the epitope, usually at least about 10 minutes. The antibody may be labeled with radioisotopes, enzymes, fluorescers, chemiluminescers, or other labels for direct detection. Alternatively, a second stage antibody or reagent is used to amplify the signal. Such reagents are well known in the art. For example, the primary antibody may be conjugated to biotin, with horseradish peroxidase-conjugated avidin added as a second stage reagent. Alternatively, the secondary antibody conjugated to a flourescent compound, *e.g*. fluorescein, rhodamine, Texas red, *etc.* Final detection uses a substrate that undergoes a color change in the presence of the peroxidase. The absence or presence of antibody binding may be determined by various methods, including flow cytometry of dissociated cells, microscopy, radiography, scintillation counting, *etc*.

Diagnostic screening may also be performed for polymorphisms that are genetically linked to a disease predisposition, particularly through the use of microsatellite markers or single nucleotide polymorphisms. Frequently the microsatellite polymorphism itself is not phenotypically expressed, but is linked to sequences that result in a disease predisposition. However, in some cases the microsatellite sequence itself may affect gene expression. Microsatellite linkage analysis may be performed alone, or in combination with direct detection of polymorphisms, as described above. The use of microsatellite markers for genotyping is well documented. For examples, see Mansfield et al. (1994), Genomics 24:225-233; Ziegle et al. (1992), Genomics 14:1026-1031; Dib *et al., supra.*

### MODULATION OF LGR4 GENE EXPRESSION

The *LGR4* genes, gene fragments, or the LGR4 protein or protein fragments, are useful in gene therapy to treat disorders associated with *LGR4* defects. Expression vectors may be used to introduce the *LGR4* gene into a cell. Such vectors generally have convenient restriction sites located near the promoter sequence to provide for the insertion of nucleic acid sequences. Transcription cassettes may be prepared comprising a transcription initiation region, the target gene or fragment thereof, and a transcriptional termination region. The transcription cassettes may be introduced into a variety of vectors, *e.g*. plasmid; retrovirus, *e.g*. lentivirus; adenovirus; and the like, where the vectors are able to transiently or stably be maintained in the cells, usually for a period of at least about one day, more usually for a period of at least about several days to several weeks.

The gene or LGR4 protein may be introduced into tissues or host cells by any number of routes, including viral infection, microinjection, or fusion of vesicles. Jet injection may also be used for intramuscular administration, as described by Furth et al. (1992), Anal Biochem 205:365-368. The DNA may be coated onto gold microparticles, and delivered intradermally by a particle bombardment device, or "gene gun" as described in the literature (see, for example, Tang et al. (1992), Nature 356:152-154), where gold microprojectiles are coated with the *LGR4, LGR5* or *LGR7* DNA, then bombarded into skin cells.

Antisense molecules can be used to down-regulate expression of *LGR4* in cells. The anti-sense reagent may be antisense oligonucleotides (ODN), particularly synthetic ODN having chemical modifications from native nucleic acids, or nucleic acid constructs that express such anti-sense molecules as RNA. The antisense sequence is complementary to the mRNA of the targeted gene, and inhibits expression of the targeted gene products. Antisense molecules inhibit gene expression through various mechanisms, *e.g*. by reducing the amount of mRNA available for translation, through activation of RNAse H, or steric hindrance. One or a combination of antisense molecules may be administered, where a combination may comprise multiple different sequences.

Antisense molecules may be produced by expression of all or a part of the target gene sequence in an appropriate vector, where the transcriptional initiation is oriented such that an antisense strand is produced as an RNA molecule. Alternatively, the antisense molecule is a synthetic oligonucleotide. Antisense oligonucleotides will generally be at least about 7, usually at least about 12, more usually at least about 20 nucleotides in length, and not more than about 500, usually not more than about 50, more usually not more than about 35 nucleotides in length, where the length is governed by efficiency of inhibition, specificity, including absence of cross-reactivity, and the like. It has been found that short oligonucleotides, of from 7 to 8 bases in length, can be strong and selective inhibitors of gene expression (see Wagner et al. (1996), Nature Biotechnol. 14:840-844).

A specific region or regions of the endogenous sense strand mRNA sequence is chosen to be complemented by the antisense sequence. Selection of a specific sequence for the oligonucleotide may use an empirical method, where several candidate sequences are assayed for inhibition of expression of the target gene in an *in vitro* or animal model. A combination of sequences may also be used, where several regions of the mRNA sequence are selected for antisense complementation.

Antisense oligonucleotides may be chemically synthesized by methods known in the art (see Wagner *et al.* (1993), *supra,* and Milligan *et al., supra.*) Preferred oligonucleotides are chemically modified from the native phosphodiester structure, in order to increase their intracellular stability and binding affinity. A number of such modifications have been described in the literature, which alter the chemistry of the backbone, sugars or heterocyclic bases.

Among useful changes in the backbone chemistry are phosphorothioates; phosphorodithioates, where both of the non-bridging oxygens are substituted with sulfur; phosphoroamidites; alkyl phosphotriesters and boranophosphates. Achiral phosphate derivatives include 3'-O'-5'-S-phosphorothioate, 3'-S-5'-O-phosphorothioate, 3'-CH₂-5'-O-phosphonate and 3'-NH-5'-O-phosphoroamidate. Peptide nucleic acids replace the entire ribose phosphodiester backbone with a peptide linkage. Sugar modifications are also used to enhance stability and affinity. The α-anomer of deoxyribose may be used, where the base is inverted with respect to the natural β-anomer. The 2'-OH of the ribose sugar may be altered to form 2'-O-methyl or 2'-O-allyl sugars, which provides resistance to degradation without comprising affinity. Modification of the heterocyclic bases must maintain proper base pairing. Some useful substitutions include deoxyuridine for deoxythymidine; 5-methyl-2'-deoxycytidine and 5-bromo-2'-deoxycytidine for deoxycytidine. 5- propynyl-2'-deoxyuridine and 5-propynyl-2'-deoxycytidine have been shown to increase affinity and biological activity when substituted for deoxythymidine and deoxycytidine, respectively.

As an alternative to anti-sense inhibitors, catalytic nucleic acid compounds, *e.g*. ribozymes, anti-sense conjugates, *etc.* may be used to inhibit gene expression. Ribozymes may be synthesized *in vitro* and administered to the patient, or may be encoded on an expression vector, from which the ribozyme is synthesized in the targeted cell (for example, see International patent application WO 9523225, and Beigelman et al. (1995), Nucl. Acids Res. 23:4434-42). Examples of oligonucleotides with catalytic activity are described in WO 9506764. Conjugates of anti-sense ODN with a metal complex, *e.g*. terpyridylCu(II), capable of mediating mRNA hydrolysis are described in Bashkin et al. (1995), Appl. Biochem. Biotechnol. 54:43-56.

### GENETICALLY ALTERED CELL OR NON-HUMAN ANIMAL MODELS FOR LGR4 FUNCTION

The subject nucleic acids can be used to generate transgenic, non-human animals or site specific gene modifications in cell lines. Transgenic animals may be made through homologous recombination, where the normal *LGR4* locus is altered. Alternatively, a nucleic acid construct is randomly integrated into the genome. Vectors for stable integration include plasmids, retroviruses and other animal viruses, YACs, and the like.

The modified cells or animals are useful in the study of *LGR4* function and regulation. For example, a series of small deletions and/or substitutions may be made in the host's native *LGR4* gene to determine the role of different exons. Of interest is the use of *LGR4* to construct transgenic animal models for disease states. Specific constructs of interest include anti-sense *LGR4* which will block LGR4 expression, expression of dominant negative *LGR4* mutations, and over-expression of *LGR4* genes. Where an *LGR4* sequence is introduced, the introduced sequence may be either a complete or partial sequence of an *LGR4* gene native to the host, or may be a complete or partial *LGR4* sequence that is exogenous to the host animal, *e.g.,* a human *LGR4* sequence. A detectable marker, such as *lac Z* may be introduced into the *LGR4* locus, where upregulation of *LGR4* expression will result in an easily detested change in phenotype.

One may also provide for expression of the *LGR4* gene or variants thereof in cells or tissues where it is not normally expressed, at levels not normally present in such cells or tissues, or at abnormal times of development. By providing expression of LGR4 protein in cells in which it is not normally produced, one can induce changes in cell behavior, *e.g*. through LGR4 mediated activity.

DNA constructs for homologous recombination will comprise at least a portion of the LGR4 gene, which may or may not be native to the species of the host animal, wherein the gene has the desired genetic modification(s), and includes regions of homology to the target locus. DNA constructs for random integration need not include regions of homology to mediate recombination. Conveniently, markers for positive and negative selection are included. Methods for generating cells having targeted gene modifications through homologous recombination are known in the art. For various techniques for transfecting mammalian cells, see Keown et al. (1990), Meth. Enzymol. 185:527-537.

For embryonic stem (ES) cells, an a non-human ES cell line may be employed, or embryonic cells may be obtained freshly from a non-human host, *e.g*. mouse, rat, guinea pig, *etc*. Such cells are grown on an appropriate fibroblast-feeder layer or grown in the presence of leukemia inhibiting factor (LIF). When ES or embryonic ceils have been transformed, they may be used to produce transgenic animals. After transformation, the cells are plated onto a feeder layer in an appropriate medium. Cells containing the construct may be detected by employing a selective medium. After sufficient time for colonies to grow, they are picked and analyzed for the occurrence of homologous recombination or integration of the construct. Those colonies that are positive may then be used for embryo manipulation and blastocyst injection. Blastocysts are obtained from 4 to 6 week old superovulated females. The ES cells are trypsinized, and the modified cells are injected into the blastocoel of the blastocyst. After injection, the blastocysts are returned to each uterine hom of pseudopregnant females. Females are then allowed to go to term and the resulting offspring screened for the construct. By providing for a different phenotype of the blastocyst and the genetically modified cells, chimeric progeny can be readily detected.

The chimeric animals are screened for the presence of the modified gene and males and females having the modification are mated to produce homozygous progeny. If the gene alterations cause lethality at some point in development, tissues or organs can be maintained as allogeneic or congenic grafts or transplants, or in *in vitro* culture. The transgenic animals may be any non-human mammal, such as laboratory animals, domestic animals, etc. The transgenic animals may be used in functional studies, drug screening, *etc., e.g.* to determine the effect of a candidate drug on *LGR4* or related gene activation *etc.*

### IN VITRO MODELS FOR LGR4 FUNCTION

The availability of a number of components in the G-protein coupled receptor family, as previously described, allows *in vitro* reconstruction of the processes or systems in which members of this family operate. Two or more of the components, such as the isolated receptor and a potential ligand therefore, may be combined *in vitro,* and the behavior assessed in terms of activation of transcription of specific target sequences; modification of protein components, *e.g*. proteolytic processing, phosphorylation, methylation, *etc.;* ability of different protein components to bind to each other. The components may be modified by sequence deletion, substitution, *etc.* to determine the functional role of specific domains.

Drug screening may be performed using an *in vitro* model, a genetically altered cell or animal, purified LGR4 protein, as well as fragments or portions thereof, e.g. solubilized extra-cellular domain or chimeric receptor proteins comprising the LGR4 extra-cellular domain. One can identify ligands or substrates that bind to and modulate the action of LGR4. Areas of investigation include the development of agents that beneficially counter abnormalities related to LGR4 and the use of such agents in the therapy.

Drug screening identifies agents that modulate the activity of LGR4 function in abnormal cells. Of particular interest are screening assays for agents that have a low toxicity for human cells. A wide variety of assays may be used for this purpose, including labeled *in vitro* protein-protein binding assays, electrophoretic mobility shift assays, immunoassays for protein binding, and the like. The purified protein may also be used for determination of three-dimensional crystal structure, which can be used for modeling intermolecular interactions, such as GTP binding, *etc.*

The term "agent" as used herein describes any molecule, *e.g*. protein or pharmaceutical, with the capability of altering or mimicking the physiological function of LGR4. Generally a plurality of assay mixtures are run in parallel with different agent concentrations to obtain a differential response to the various concentrations. Typically, one of these concentrations serves as a negative control, *i.e*. at zero concentration or below the level of detection.

In some embodiments, candidate agents encompass numerous chemical classes, though typically they are organic molecules, preferably small organic compounds having a molecular weight of more than 50 and less than about 2,500 daltons. Candidate agents comprise functional groups necessary for structural interaction with proteins, particularly hydrogen bonding, and typically include at least an amine, carbonyl, hydroxyl or carboxyl group, preferably at least two of the functional chemical groups. The candidate agents often comprise cyclical carbon or heterocyclic structures and/or aromatic or polyaromatic structures substituted with one or more of the above functional groups. Candidate agents are also found among biomolecules including peptides, saccharides, fatty acids, steroids, purines, pyrimidines, derivatives, structural analogs or combinations thereof.

Candidate agents are obtained from a wide variety of sources including libraries of synthetic or natural compounds. For example, numerous means are available for random and directed synthesis of a wide variety of organic compounds and biomolecules, including expression of randomized oligonucleotides and oligopeptides. Alternatively, libraries of natural compounds in the form of bacterial, fungal, plant and animal extracts are available or readily produced. Additionally, natural or synthetically produced libraries and compounds are readily modified through conventional chemical, physical and biochemical means, and may be used to produce combinatorial libraries. Known pharmacological agents may be subjected to directed or random chemical modifications, such as acylation, alkylation, esterification, amidification, etc. to produce structural analogs.

Of particular interest in certain embodiments are peptidic agents based on LGR4, e.g. solubilized extra-cellular domain or chimeric receptor proteins comprising the LGR4 extra-cellular domain, where such agents neutralize the activity of endogenous LGR4 ligands, e.g. hormones.

Where the screening assay is a binding assay, one or more of the molecules may be joined to a label, where the label can directly or indirectly provide a detectable signal. Various labels include radioisotopes, fluorescers, chemiluminescers, enzymes, specific binding molecules, particles, *e.g*. magnetic particles, and the like. Specific binding molecules include pairs, such as biotin and streptavidin, digoxin and antidigoxin etc. For the specific binding members, the complementary member would normally be labeled with a molecule that provides for detection, in accordance with known procedures.

A variety of other reagents may be included in the screening assay. These include reagents like salts, neutral proteins, e.g. albumin, detergents, etc., that are used to facilitate optimal protein-protein binding and/or reduce non-specific or background interactions. Reagents that improve the efficiency of the assay, such as protease inhibitors, nuclease inhibitors, anti-microbial agents, *etc*., may be used. The mixture of components are added in any order that provides for the requisite binding. Incubations are performed at any suitable temperature, typically between 4 and 40°C. Incubation periods are selected for optimum activity, but may also be optimized to facilitate rapid high-throughput screening. Typically between 0.1 and 1 hours will be sufficient.

Other assays of interest detect agents that mimic LGR4 function. For example, an expression construct comprising an *LGR4* gene may be introduced into a cell line under conditions that allow expression. The level of LGR4 activity is determined by a functional assay, as previously described. In one screening assay, the ability of candidate agents to inhibit or enhance LGR4 function is determined. Alternatively, candidate agents are added to a cell that lacks functional LGR4, and screened for the ability to reproduce LGR4 activity in a functional assay.

The compounds having the desired pharmacological activity may be administered in a physiologically acceptable carrier to a host for treatment, etc. The compounds may also be used to enhance *LGR4* function. The inhibitory agents may be administered in a variety of ways, orally, topically, parenterally *e.g*. subcutaneously, intraperitoneally, by viral infection, intravascularly, *etc*. Topical treatments are of particular interest. Depending upon the manner of introduction, the compounds may be formulated in a variety of ways. The concentration of therapeutically active compound in the formulation may vary from about 0.1-100 wt.%.

The pharmaceutical compositions can be prepared in various forms, such as granules, tablets, pills, suppositories, capsules, suspensions, salves, lotions and the like. Pharmaceutical grade organic or inorganic carriers and/or diluents suitable for oral and topical use can be used to make up compositions containing the therapeutically-active compounds. Diluents known to the art include aqueous media, vegetable and animal oils and fats. Stabilizing agents, wetting and emulsifying agents, salts for varying the osmotic pressure or buffers for securing an adequate pH value, and skin penetration enhancers can be used as auxiliary agents.

### EXPERIMENTAL

The following examples are put forth so as to provide those of ordinary skill in the art with a complete disclosure and description of how to make and use the subject invention, and are not intended to limit the scope of what is regarded as the invention. Efforts have been made to ensure accuracy with respect to the numbers used (*e.g.* amounts, temperature, concentrations, *etc*.) but some experimental errors and deviations should be allowed for. Unless otherwise indicated, parts are parts by weight, molecular weight is average molecular weight, temperature is in degrees centigrade; and pressure is at or near atmospheric.

### Example 1. Identification of LGR4 and LGR5

Human sequences related to the sea anemone and Drosophila glycoprotein hormone receptors were identified from the expression sequence tag database (dbEST) at the National Center for Biotechnology Information by using the BLAST server with the BLOSUM62 protein comparison matrix (Altschul SF et al, Nucleic Acids Res (1997) 25:3389-3402). Human ESTs showing high homology to two non-overlapping regions of the gonadotropin receptors were identified. Clones AA312798 and AA298810 were found to encode transmembrane four to five of the putative receptor LGR4 whereas AA460529 and AA424098 encode transmembrane two to three of the putative receptor LGR5. Using these ESTs to further search the GenBank EST division database, overlapping EST sequences were aligned to obtain the longest open reading frame (ORF) for these receptors.

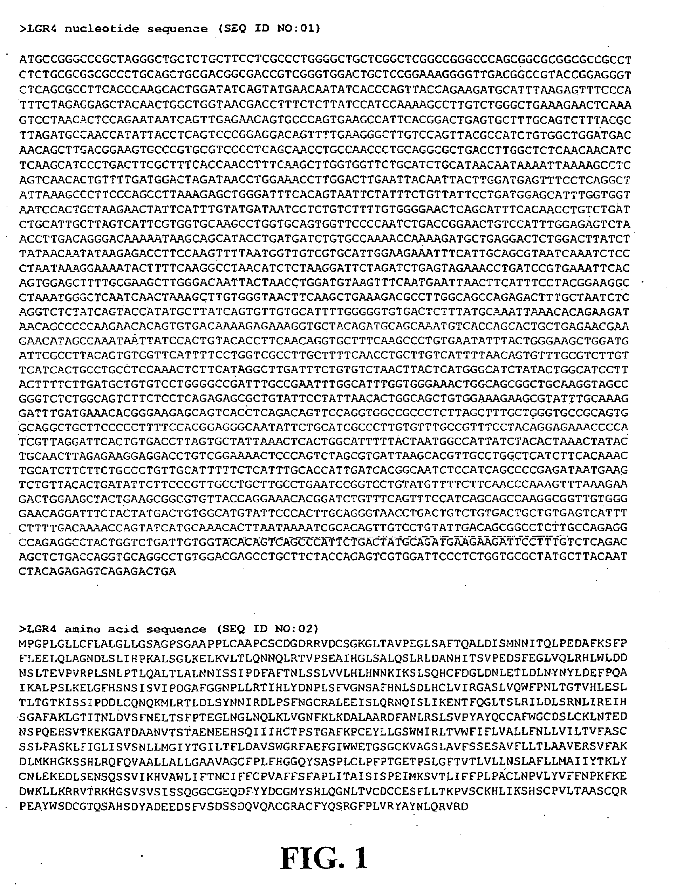

Based on the longest human ORF, specific primers were designed for PCR amplification of LGR4 and LGR5 cDNA fragments from rat ovary and human placenta, respectively. After hybridization with labeled EST clones and confirmation of DNA sequences by dideoxy DNA sequencing, specific receptor fragments isolated were used to design primers to prepare sub-cDNA libraries enriched with specific receptor cDNAs. For 5' extension, reverse transcription was performed using rat ovarian and human placenta mRNA preparations and receptor-specific primers. Following second strand synthesis, the enriched cDNA pool was tailed at 5'-ends with specific adaptor sequences to allow further PCR amplification. For 3' extension, rat ovarian or human placenta mRNAs were reversed transcribed using oligo-dT, followed by second strand synthesis using receptor-specific primers and adaptor tailing. These mini-libraries were further used as templates for PCR amplification of upstream or downstream cDNAs specific for each receptor using internal primers. PCR products with a strong hybridization signal to each receptor cDNA fragment were subcloned into the pUC18 or pcDNA3 vectors. After screening of these sublibraries based on colony hybridization using specific receptor probes, clones with 5'-or 3'-sequences of the putative receptors were identified and isolated for DNA sequencing. As needed, the procedure was repeated up to three times to generate cDNAs encoding the complete ORF of each putative receptor for sequence analysis and for the expression of receptor proteins in eukaryotic cells. The entire coding sequences of each gene were also amplified with specific primers flanking the entire ORF in independent experiments. At least three independent PCR clones were sequenced to verify the authenticity of coding sequences. The nucleotide sequence of LGR4, as well as the amino acid sequence of the product encoded by the ORF thereof, is provided in Fig. 1. The nucleotide sequence of LGR5, as well as the amino acid sequence of the product encoded by the ORF thereof, is provided in SEQ ID NO : 3 and SEQ ID NO:4

### Example 2. Comparison of deduced amino acid sequence of LGR4 and 5 cDNAs and those encoding FSH and LH receptors.

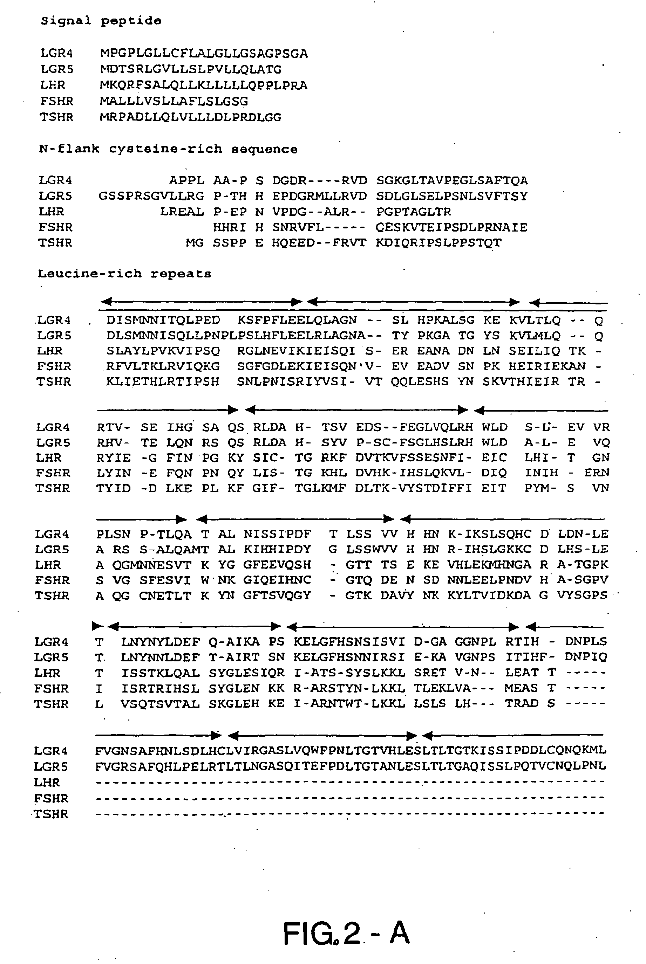

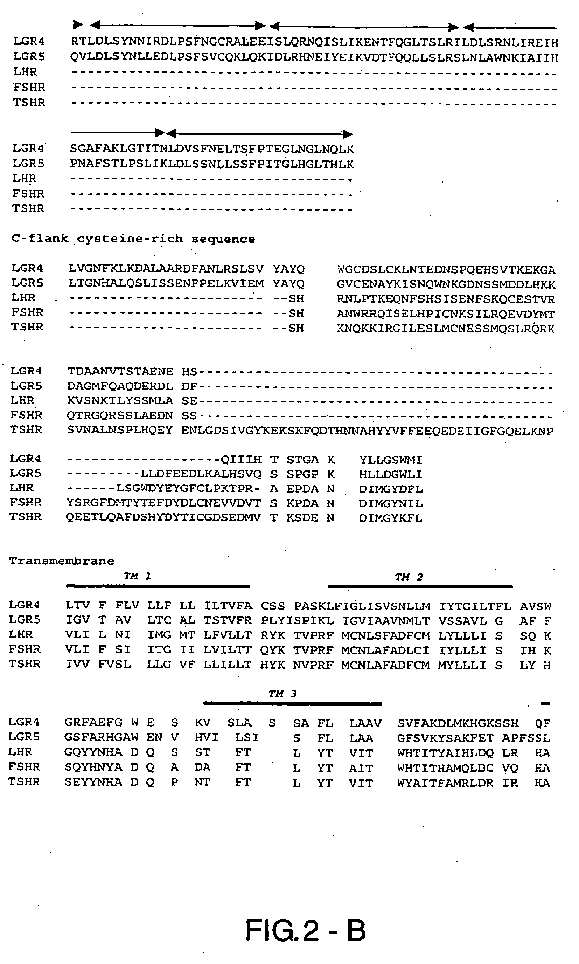

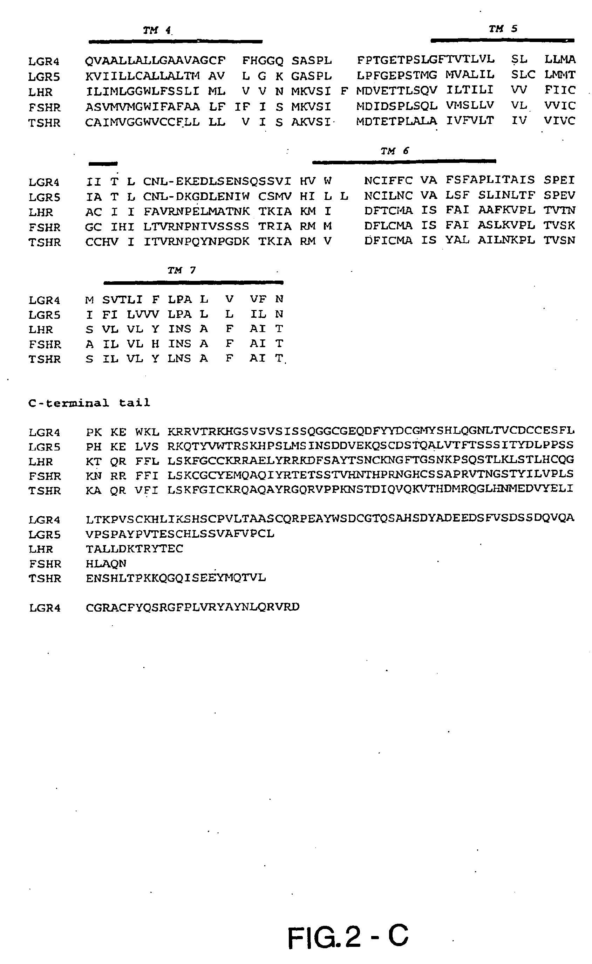

Sequence alignment of LGR4 and LGR5 with known human glycoprotein hormone receptors was performed and the results are shown in Fig. 2. Shaded residues are identical in at least two of the four receptor proteins shown.

### Example 3. Expression pattern of LGR4 and 5 mRNA transcripts in different tissues.

For northern blot analysis, poly (A)+-selected RNA from different human tissues was hybridized with a ³²P-labeled cDNA probes. After washing, the blots were exposed to X-ray films at -70C for five days. Subsequent hybridization with a beta-actin cDNA probe was performed to estimate nucleic acid loading (8 h exposure). LGR4 was shown to be expressed in placenta, ovary, testis, adrenal, spinal cord, thyroid, stomach, trachea, heart, pancreas, kidney, prostate and spleen while LGR5 was shown to be expressed in the skeletal muscle, placenta, spinal cord, brain, adrenal, colon, stomach, ovary and bone marrow.

### Example 4. Chromosomal localization of LGR4 and 5 in human.

Using genomic fragments of LGR4 (> 100 Kb) and LGR5 (> 100 Kb) as probes, chromosomal localization of these genes were detected using the FISH method to banded DNA in chromosomal 5q34-35.1 and 12q15, respectively.

It is evident from the above discussion and results that LGR4 mammalian G-protein coupled receptors, as well as a nucleic acids encoding the same, are provided by the subject invention. The inventions described above find use in a variety of applications, including research and therapeutic applications.

The publications discussed herein are provided solely for their disclosure prior to the filing date of the present application.

## Claims

1. An isolated nucleic acid encoding a mammalian protein, wherein said mammalian protein comprises an amino acid sequence with at least 80% sequence identity to SEQ ID NO: 2.

2. The isolated nucleic acid according to Claim 1, wherein said mammalian protein has an amino acid sequence with at least 90% sequence identity to SEQ ID NO: 2.

3. The isolated nucleic acid according to Claim 1, wherein said mammalian protein has an amino acid sequence with at least 98% sequence identity to SEQ ID NO: 2.

4. An isolated nucleic acid according to Claim 1, wherein said mammalian protein has the amino acid sequence of SEQ ID NO: 2.

5. An isolated nucleic acid, wherein the nucleotide sequence of said nucleic acid comprises a nucleotide sequence having at least 75% sequence identity to the sequence set forth in SEQ ID NO: 1 or the complementary sequence thereof.

6. The isolated nucleic acid according to claim 5, wherein the nucleotide sequence of said nucleic acid comprises a nucleotide sequence having at least 90% sequence identity to the sequence set forth in SEQ ID NO: 1 or the complementary sequence thereof.

7. The isolated nucleic acid according to claim 5, wherein the nucleotide sequence of said nucleic acid comprises a nucleotide sequence having at least 95% sequence identity to the sequence set forth in SEQ ID NO: 1 or the complementary sequence thereof.

8. An isolated nucleic acid comprising at least 50 contiguous nucleotides of the sequence of SEQ ID NO: 1 or the complementary sequence thereof.

9. An expression cassette comprising a transcriptional initiation region functional in an expression host, a nucleic acid having a sequence of the isolated nucleic acid according to Claim 1 under the transcriptional regulation of said transcriptional initiation region, and a transcriptional termination region functional in said expression host.

10. An *in vitro* cell comprising an expression cassette according to Claim 9 as part of an extrachromosomal element or integrated into the genome of said cell as a result of introduction of said expression cassette into said cell.

11. A non-human cell comprising an expression cassette according to Claim 9 as part of an extrachromosomal element or integrated into the genome of said cell as a result of introduction of said expression cassette into said cell.

12. The cellular progeny of a cell according to Claim 10 or 11, wherein said cellular progeny comprises said expression cassette as part of an extrachromosomal element or integrated into the genome of said cellular progeny.

13. A method for producing a mammalian protein comprising an amino acid sequence with at least 80% sequence identity to SEQ ID NO: 2, said method comprising:
growing a cell according to any one of claims 10 to 12, whereby said mammalian protein is expressed; and
isolating said protein substantially free of other proteins.

14. A purified polypeptide composition comprising at least 50 weight % of the protein present as a mammalian protein, wherein said mammalian protein comprises an amino acid sequence with at least 80% sequence identity to SEQ ID NO: 2.

15. An antibody that binds specifically to a polypeptide comprising the amino acid sequence set forth in SEQ ID NO:2.

16. The antibody of Claim 15, wherein said antibody is a monoclonal antibody.

17. A non-human transgenic animal, wherein said animal in its native form comprises a gene encoding an amino acid sequence having at least 80% sequence identity to SEQ ID NO:2, and said gene is modified in the transgenic animal by an introduced nucleic acid insertion or deletion in said gene.

18. The non-human transgenic animal of claim 17, wherein said animal is heterozygous for said introduced nucleic acid insertion or deletion.

19. The non-human transgenic animal of claim 17, wherein said animal is homozygous for said introduced nucleic acid insertion or deletion.

20. The non-human transgenic animal of claim 17, wherein said introduced nucleic acid insertion or deletion is a knockout of endogenous expression of a gene encoding a mammalian protein comprising an amino acid sequence having at least 80% sequence identity to SEQ ID NO: 2.

21. A method of screening a sample for the presence of a ligand for a receptor, wherein said receptor is a mammalian protein comprising an amino acid sequence having at least 80% sequence identity to SEQ ID NO: 2 said method comprising:
contacting said sample with a receptor comprising an amino acid sequence with at least 80% sequence identity to SEQ ID NO: 2, and
detecting the presence of a binding event between said receptor and ligand in said sample.

## Patentansprüche

1. Isolierte Nucleinsäure, die für ein Säugetierprotein kodiert, worin das Säugetierprotein eine Aminosäuresequenz mit zumindest 80 % Sequenzidentität zu Seq.-ID Nr. 2 umfasst.

2. Isolierte Nucleinsäure nach Anspruch 1, worin das Säugetierprotein eine Aminosäuresequenz mit zumindest 90 % Sequenzidentität zu Seq.-ID Nr. 2 aufweist.

3. Isolierte Nucleinsäure nach Anspruch 1, worin das Säugetierprotein eine Aminosäuresequenz mit zumindest 98 % Sequenzidentität zu Seq.-ID Nr. 2 aufweist.

4. Isolierte Nucleinsäure nach Anspruch 1, worin das Säugetierprotein die Aminosäuresequenz von Seq.-ID Nr. 2 aufweist.

5. Isolierte Nucleinsäure, worin die Nucleotidsequenz der Nucleinsäure eine Nucleotidsequenz mit zumindest 75 % Sequenzidentität zu der in Seq.-ID Nr. 1 gezeigten Sequenz oder der Komplementärsequenz dazu umfasst.

6. Isolierte Nucleinsäure nach Anspruch 5, worin die Nucleotidsequenz der Nucleinsäure eine Nucleotidsequenz mit zumindest 90 % Sequenzidentität zu der in Seq.-ID Nr. 1 gezeigten Sequenz oder der Komplementärsequenz dazu umfasst.

7. Isolierte Nucleinsäure nach Anspruch 5, worin die Nucleotidsequenz der Nucleinsäure eine Nucleotidsequenz mit zumindest 95 % Sequenzidentität zu der in Seq.-ID Nr. 1 gezeigten Sequenz oder der Komplementärsequenz dazu umfasst.

8. Isolierte Nucleinsäure, die zumindest 50 zusammenhängende Nucleotide der Sequenz von Seq.-ID Nr. 1 oder der Komplementärsequenz dazu umfasst.

9. Expressionskassette, die eine in einem Expressionswirt funktionelle Transkriptionsinitiationsregion, eine Nucleinsäure mit einer Sequenz der isolierten Nucleinsäure nach Anspruch 1 unter der Transkriptionsregulation der Transkriptionsinitiationsregion sowie eine in dem Expressionswirt funktionelle Transkriptionsterminationsregion umfasst.

10. In-vitro-Zelle, die eine Expressionskassette nach Anspruch 9 als Teil eines extrachromosomalen Elements oder als Ergebnis einer Einführung der Expressionskassette in die Zelle in das Genom der Zelle integriert umfasst.

11. Nichtmenschliche Zelle, die eine Expressionskassette nach Anspruch 9 als Teil eines extrachromosomalen Elements oder als Ergebnis einer Einführung der Expressionskassette in die Zelle in das Genom der Zelle integriert umfasst.

12. Zellnachkommenschaft einer Zelle nach Anspruch 10 oder 11, worin die Zellnachkommenschaft die Expressionskassette als Teil eines extrachromosomalen Elements oder in das Genom der Zellnachkommenschaft integriert umfasst.

13. Verfahren zum Produzieren eines Säugetierproteins, das eine Aminosäuresequenz mit zumindest 80 % Sequenzidentität zu Seq.-ID Nr. 2 umfasst, wobei das Verfahren Folgendes umfasst:
Züchten einer Zelle nach einem der Ansprüche 10 bis 12, wodurch das Säugetierprotein exprimiert wird; und
Isolieren des Proteins, so dass es im Wesentlichen frei von anderen Proteinen ist.

14. Gereinigte Polypeptid-Zusammensetzung, die zumindest 50 Gew.-% des Proteins umfasst, das als Säugetierprotein vorliegt, worin das Säugetierprotein eine Aminosäuresequenz mit zumindest 80 % Sequenzidentität zu Seq.-ID Nr. 2 umfasst.

15. Antikörper, der spezifisch an ein Polypeptid bindet, das die in Seq.-ID Nr. 2 gezeigte Aminosäuresequenz umfasst.

16. Antikörper nach Anspruch 15, worin der Antikörper ein monoklonaler Antikörper ist.

17. Nichtmenschliches transgenes Tier, worin das Tier in seiner nativen Form ein Gen umfasst, das für eine Aminosäure mit zumindest 80 % Sequenzidentität zu Seq.-ID Nr. 2 kodiert, und das Gen in dem transgenen Tier durch eine eingeführte Nucleinsäureinsertion oder -deletion in das Gen modifiziert ist.

18. Nichtmenschliches transgenes Tier nach Anspruch 17, worin das Tier bezüglich der eingeführten Nucleinsäureinsertion oder -deletion heterozygot ist.

19. Nichtmenschliches transgenes Tier nach Anspruch 17, worin das Tier bezüglich der eingeführten Nucleinsäureinsertion oder -deletion homozygot ist.

20. Nichtmenschliches transgenes Tier nach Anspruch 17, worin die eingeführte Nucleinsäureinsertion oder -deletion einen Knockout der endogenen Expression eines Gens darstellt, das für ein Säugetierprotein kodiert, das eine Aminosäuresequenz mit zumindest 80 % Sequenzidentität zu Seq.-ID Nr. 2 umfasst.

21. Verfahren zum Screenen einer Probe auf die Gegenwart eines Liganden für einen Rezeptor, worin der Rezeptor ein Säugetierprotein ist, das eine Aminosäuresequenz mit zumindest 80 % Sequenzidentität zu Seq.-ID Nr. 2 umfasst, wobei das Verfahren Folgendes umfasst:
Kontaktieren der Probe mit einem Rezeptor, der eine Aminosäuresequenz mit zumindest 80 % Sequenzidentität zu Seq.-ID Nr. 2 umfasst, und
Nachweisen der Gegenwart einer Bindung zwischen dem Rezeptor und dem Liganden in der Probe.

## Revendications

1. Acide nucléique isolé codant pour une protéine mammalienne, où ladite protéine mammalienne comprend une séquence d'acides aminés ayant au moins 80 % d'identité de séquence avec SEQ ID NO: 2.

2. Acide nucléique isolé selon la revendication 1, où ladite protéine mammalienne possède une séquence d'acides aminés ayant au moins 90 % d'identité de séquence avec SEQ ID NO: 2.

3. Acide nucléique isolé selon la revendication 1, où ladite protéine mammalienne possède une séquence d'acides aminés ayant au moins 98 % d'identité de séquence avec SEQ ID NO: 2.

4. Acide nucléique isolé selon la revendication 1, où ladite protéine mammalienne possède la séquence d'acides aminés de SEQ ID NO: 2.

5. Acide nucléique isolé, où la séquence de nucléotides dudit acide nucléique comprend une séquence de nucléotides ayant au moins 75 % d'identité de séquence avec la séquence décrite dans SEQ ID NO: 1 ou la séquence complémentaire de celle-ci.

6. Acide nucléique isolé selon la revendication 5, où la séquence de nucléotides dudit acide nucléique comprend une séquence de nucléotides ayant au moins 90 % d'identité de séquence avec la séquence décrite dans SEQ ID NO: 1 ou la séquence complémentaire de celle-ci.

7. Acide nucléique isolé selon la revendication 5, où la séquence de nucléotides dudit acide nucléique comprend une séquence de nucléotides ayant au moins 95 % d'identité de séquence avec la séquence décrite dans SEQ ID NO: 1 ou la séquence complémentaire de celle-ci.

8. Acide nucléique isolé comprenant au moins 50 nucléotides contigus de la séquence de SEQ ID NO: 1 ou la séquence complémentaire de celle-ci.

9. Cassette d'expression comprenant une région d'initiation de la transcription qui est fonctionnelle dans un hôte d'expression, un acide nucléique ayant une séquence de l'acide nucléique isolé selon la revendication 1, sous la régulation transcriptionnelle de ladite région d'initiation de la transcription, et une région de terminaison de la transcription qui est fonctionnelle dans ledit hôte d'expression.

10. Cellule *in vitro* comprenant une cassette d'expression selon la revendication 9 en tant que partie d'un élément extrachromosomique ou intégrée dans le génome de ladite cellule en conséquence de l'introduction de ladite cassette d'expression dans ladite cellule.

11. Cellule non humaine comprenant une cassette d'expression selon la revendication 9 en tant que partie d'un élément extrachromosomique ou intégrée dans le génome de ladite cellule en conséquence de l'introduction de ladite cassette d'expression dans ladite cellule.

12. Descendance cellulaire d'une cellule selon la revendication 10 ou 11, où ladite descendance cellulaire comprend ladite cassette d'expression en tant que partie d'un élément extrachromosomique ou intégrée dans le génome de ladite descendance cellulaire.

13. Procédé pour produire une protéine mammalienne comprenant une séquence d'acides aminés ayant au moins 80 % d'identité de séquence avec SEQ ID NO: 2, ledit procédé consistant à:
cultiver une cellule selon l'une quelconque des revendications 10 à 12, moyennant quoi ladite protéine mammalienne est exprimée; et
isoler ladite protéine essentiellement exempte d'autres protéines.

14. Composition polypeptidique purifiée comprenant au moins 50 % en poids de la protéine présente en tant que protéine mammalienne, où ladite protéine mammalienne comprend une séquence d'acides aminés ayant au moins 80 % d'identité de séquence avec SEQ ID NO: 2.

15. Anticorps qui se lie spécifiquement à un polypeptide comprenant la séquence d'acides aminés décrite dans SEQ ID NO: 2.

16. Anticorps selon la revendication 15, où ledit anticorps est un anticorps monoclonal.

17. Animal transgénique non humain, où ledit animal, sous sa forme native, comprend un gène codant pour une séquence d'acides aminés ayant au moins 80 % d'identité de séquence avec SEQ ID NO: 2, et ledit gène est modifié chez l'animal transgénique par une insertion ou une délétion d'acide nucléique introduite dans ledit gène.

18. Animal transgénique non humain selon la revendication 17, où ledit animal est hétérozygote pour ladite insertion ou délétion d'acide nucléique introduite.

19. Animal transgénique non humain selon la revendication 17, où ledit animal est homozygote pour ladite insertion ou délétion d'acide nucléique introduite.

20. Animal transgénique non humain selon la revendication 17, où ladite insertion ou délétion d'acide nucléique introduite est un knock-out de l'expression endogène d'un gène codant pour une protéine mammalienne comprenant une séquence d'acides aminés ayant au moins 80 % d'identité de séquence avec SEQ ID NO: 2.

21. Procédé de criblage d'un échantillon pour la présence d'un ligand à un récepteur, où ledit récepteur est une protéine mammalienne comprenant une séquence d'acides aminés ayant au moins 80 % d'identité de séquence avec SEQ ID NO: 2, ledit procédé consistant à:
mettre en contact ledit échantillon avec un récepteur comprenant une séquence d'acides aminés ayant au moins 80 % d'identité de séquence avec SEQ ID NO: 2, et
détecter la présence d'un événement de liaison entre ledit récepteur et le ligand dans ledit échantillon.


### SEQUENCE LISTING

<110> Hsueh, Aaron
   Hsu, Yu Sheau
   Liang, Shan-Guang
   van der Spek, Petrus Johannes
<120> Novel Mammalian G-Protein Coupled
   Receptors Having Extracellular Leucine Rich Repeat Regions
<130> SUN-84PCT
<160> 8
<170> FastSEQ for Windows Version 3.0
<210> 1
   <211> 2856
   <212> DNA
   <213> human
<400> 1
   atgccgggcc cgctagggct gctctgcttc ctcgccctgg ggctgctcgg ctcggccggg      60
   cccagcggcg cggcgccgcc tctctgcgcg gcgccctgca gctgcgacgg cgaccgtcgg     120
   gtggactgct ccggaaaggg gttgacggcc gtaccggagg gtctcagcgc cttcacccaa     180
   gcactggata tcagtatgaa caatatcacc cagttaccag aagatgcatt taagagtttc     240
   ccatttctag aggagctaca actggctggt aacgaccttt ctcttatcca tccaaaagcc     300
   ttgtctgggc tgaaagaact caaagtccta acactccaga ataatcagtt gagaacagtg     360
   cccagtgaag ccattcacgg actgagtgct ttgcagtctt tacgcttaga tgccaaccat     420
   attacctcag tcccggagga cagttttgaa gggcttgtcc agttacgcca tctgtggctg     480
   gatgacaaca gcttgacgga agtgcccgtg cgtcccctca gcaacctgcc aaccctgcag     540
   gcgctgacct tggctctcaa caacatctca agcatccctg acttcgcttt caccaacctt     600
   tcaagcttgg tggttctgca tctgcataac aataaaatta aaagcctcag tcaacactgt     660
   tttgatggac tagataacct ggaaaccttg gacttgaatt acaattactt ggatgagttt     720
   cctcaggcta ttaaagccct tcccagcctt aaagagctgg gatttcacag taattctatt     780
   tctgttattc ctgatggagc atttggtggt aatccactgc taagaactat tcatttgtat     840
   gataatcctc tgtcttttgt ggggaactca gcatttcaca acctgtctga tctgcattgc     900
   ttagtcattc gtggtgcaag cctggtgcag tggttcccca atctgaccgg aactgtccat     960
   ttggagagtc taaccttgac agggacaaaa ataagcagca tacctgatga tctgtgccaa    1020
   aaccaaaaga tgctgaggac tctggactta tcttataaca atataagaga ccttccaagt    1080
   tttaatggtt gtcgtgcatt ggaagaaatt tcattgcagc gtaatcaaat ctccctaata    1140
   aaggaaaata cttttcaagg cctaacatct ctaaggattc tagatctgag tagaaacctg    1200
   atccgtgaaa ttcacagtgg agcttttgcg aagcttggga caattactaa cctggatgta    1260
   agtttcaatg aattaacttc atttcctacg gaaggcctaa atgggctcaa tcaactaaag    1320
   cttgtgggta acttcaagct gaaagacgcc ttggcagcca gagactttgc taatctcagg    1380
   tctctatcag taccatatgc ttatcagtgt tgtgcatttt gggggtgtga ctctttatgc    1440
   aaattaaaca cagaagataa cagcccccaa gaacacagtg tgacaaaaga gaaaggtgct    1500
   acagatgcag caaatgtcac cagcactgct gagaacgaag aacatagcca aataattatc    1560
   cactgtacac cttcaacagg tgctttcaag ccctgtgaat atttactggg aagctggatg    1620
   attcgcctta cagtgtggtt cattttcctg gtcgccttgc ttttcaacct gcttgtcatt    1680
   ttaacagtgt ttgcgtcttg ttcatcactg cctgcctcca aactcttcat aggcttgatt    1740
   tctgtgtcta acttactcat gggcatctat actggcatcc ttacttttct tgatgctgtg    1800
   tcctggggcc gatttgccga atttggcatt tggtgggaaa ctggcagcgg ctgcaaggta    1860
   gccgggtctc tggcagtctt ctcctcagag agcgctgtat tcctattaac actggcagct    1920
   gtggaaagaa gcgtatttgc aaaggatttg atgaaacacg ggaagagcag tcacctcaga    1980
   cagttccagg tggccgccct cttagctttg ctgggtgccg cagtggcagg ctgcttcccc    2040
   cttttccacg gagggcaata ttctgcatcg ccccttgttt tgccgtttcc tacaggagaa   2100
   accccatcgt taggattcac tgtgacctta gtgctattaa actcactggc atttttacta    2160
   atggccatta tctacactaa actatactgc aacttagaga aggaggacct gtcggaaaac    2220
   tcccagtcta gcgtgattaa gcacgttgcc tggctcatct tcacaaactg catcttcttc    2280
   tgccctgttg catttttctc atttgcacca ttgatcacgg caatctccat cagccccgag    2340
   ataatgaagt ctgttacact gatattcttc ccgttgcctg cttgcctgaa tccggtcctg    2400
   tatgttttct tcaacccaaa gtttaaagaa gactggaagc tactgaagcg gcgtgttacc    2460
   aggaaacacg gatctgtttc agtttccatc agcagccaag gcggttgtgg ggaacaggat    2520
   ttctactatg actgtggcat gtattcccac ttgcagggta acctgactgt ctgtgactgc    2580
   tgtgagtcat ttcttttgac aaaaccagta tcatgcaaac acttaataaa atcgcacagt    2640
   tgtcctgtat tgacagcggc ctcttgccag aggccagagg cctactggtc tgattgtggt    2700
   acacagtcag cccattctga ctatgcagat gaagaagatt cctttgtctc agacagctct    2760
   gaccaggtgc aggcctgtgg acgagcctgc ttctaccaga gtcgtggatt ccctctggtg    2820
   cgctatgctt acaatctaca gagagtcaga gactga                              2856
<210> 2
   <211> 951
   <212> PRT
   <213> human
<400> 2
   Met Pro Gly Pro Leu Gly Leu Leu Cys Phe Leu Ala Leu Gly Leu Leu
    1               5                  10                  15
   Gly Ser Ala Gly Pro Ser Gly Ala Ala Pro Pro Leu Cys Ala Ala Pro
               20                  25                  30
   Cys Ser Cys Asp Gly Asp Arg Arg Val Asp Cys Ser Gly Lys Gly Leu
           35                  40                  45
   Thr Ala Val Pro Glu Gly Leu Ser Ala Phe Thr Gln Ala Leu Asp Ile
       50                  55                  60
   Ser Met Asn Asn Ile Thr Gln Leu Pro Glu Asp Ala Phe Lys Ser Phe
   65                  70                  75                  80
   Pro Phe Leu Glu Glu Leu Gln Leu Ala Gly Asn Asp Leu Ser Leu Ile
                   85                  90                  95
   His Pro Lys Ala Leu Ser Gly Leu Lys Glu Leu Lys Val Leu Thr Leu
               100                 105                 110
   Gln Asn Asn Gln Leu Arg Thr Val Pro Ser Glu Ala Ile His Gly Leu
           115                 120                 125
   Ser Ala Leu Gln Ser Leu Arg Leu Asp Ala Asn His Ile Thr Ser Val
       130                 135                 140
   Pro Glu Asp Ser Phe Glu Gly Leu Val Gln Leu Arg His Leu Trp Leu
   145                 150                 155                 160
   Asp Asp Asn Ser Leu Thr Glu Val Pro Val Arg Pro Leu Ser Asn Leu
                   165                 170                 175
   Pro Thr Leu Gln Ala Leu Thr Leu Ala Leu Asn Asn Ile Ser Ser Ile
               180                 185                 190
   Pro Asp Phe Ala Phe Thr Asn Leu Ser Ser Leu Val Val Leu His Leu
           195                 200                 205
   His Asn Asn Lys Ile Lys Ser Leu Ser Gln His Cys Phe Asp Gly Leu
       210                 215                 220
   Asp Asn Leu Glu Thr Leu Asp Leu Asn Tyr Asn Tyr Leu Asp Glu Phe
   225                 230                 235                 240
   Pro Gln Ala Ile Lys Ala Leu Pro Ser Leu Lys Glu Leu Gly Phe His
                   245                 250                 255
   Ser Asn Ser Ile Ser Val Ile Pro Asp Gly Ala Phe Gly Gly Asn Pro
               260                 265                 270
   Leu Leu Arg Thr Ile His Leu Tyr Asp Asn Pro Leu Ser Phe Val Gly
           275                 280                 285
   Asn Ser Ala Phe His Asn Leu Ser Asp Leu His Cys Leu Val Ile Arg
       290                 295                 300
   Gly Ala Ser Leu Val Gln Trp Phe Pro Asn Leu Thr Gly Thr Val His
   305                 310                 315                 320
   Leu Glu Ser Leu Thr Leu Thr Gly Thr Lys Ile Ser Ser Ile Pro Asp
                   325                 330                 335
   Asp Leu Cys Gln Asn Gln Lys Met Leu Arg Thr Leu Asp Leu Ser Tyr
               340                 345                 350
   Asn Asn Ile Arg Asp Leu Pro Ser Phe Asn Gly Cys Arg Ala Leu Glu
           355                 360                 365
   Glu Ile Ser Leu Gln Arg Asn Gln Ile Ser Leu Ile Lys Glu Asn Thr
       370                 375                 380
   Phe Gln Gly Leu Thr Ser Leu Arg Ile Leu Asp Leu Ser Arg Asn Leu
   385                 390                 395                 400
   Ile Arg Glu Ile His Ser Gly Ala Phe Ala Lys Leu Gly Thr Ile Thr
                   405                 410                 415
   Asn Leu Asp Val Ser Phe Asn Glu Leu Thr Ser Phe Pro Thr Glu Gly
               420                 425                 430
   Leu Asn Gly Leu Asn Gln Leu Lys Leu Val Gly Asn Phe Lys Leu Lys
           435                 440                 445
   Asp Ala Leu Ala Ala Arg Asp Phe Ala Asn Leu Arg Ser Leu Ser Val
       450                 455                 460
   Pro Tyr Ala Tyr Gln Cys Cys Ala Phe Trp Gly Cys Asp Ser Leu Cys
   465                 470                 475                 480
   Lys Leu Asn Thr Glu Asp Asn Ser Pro Gln Glu His Ser Val Thr Lys
                   485                 490                 495
   Glu Lys Gly Ala Thr Asp Ala Ala Asn Val Thr Ser Thr Ala Glu Asn
               500                 505                 510
   Glu Glu His Ser Gln Ile Ile Ile His Cys Thr Pro Ser Thr Gly Ala
           515                 520                 525
   Phe Lys Pro Cys Glu Tyr Leu Leu Gly Ser Trp Met Ile Arg Leu Thr
       530                 535                 540
   Val Trp Phe Ile Phe Leu Val Ala Leu Leu Phe Asn Leu Leu Val Ile
   545                 550                 555                 560
   Leu Thr Val Phe Ala Ser Cys Ser Ser Leu Pro Ala Ser Lys Leu Phe
                   565                 570                 575
   Ile Gly Leu Ile Ser Val Ser Asn Leu Leu Met Gly Ile Tyr Thr Gly
               580                 585                 590
   Ile Leu Thr Phe Leu Asp Ala Val Ser Trp Gly Arg Phe Ala Glu Phe
           595                 600                 605
   Gly Ile Trp Trp Glu Thr Gly Ser Gly Cys Lys Val Ala Gly Ser Leu
       610                 615                 620
   Ala Val Phe Ser Ser Glu Ser Ala Val Phe Leu Leu Thr Leu Ala Ala
   625                 630                 635                 640
   Val Glu Arg Ser Val Phe Ala Lys Asp Leu Met Lys His Gly Lys Ser
                   645                 650                 655
   Ser His Leu Arg Gln Phe Gln Val Ala Ala Leu Leu Ala Leu Leu Gly
               660                 665                 670
   Ala Ala Val Ala Gly Cys Phe Pro Leu Phe His Gly Gly Gln Tyr Ser
           675                 680                 685
   Ala Ser Pro Leu Cys Leu Pro Phe Pro Thr Gly Glu Thr Pro Ser Leu
       690                 695                 700
   Gly Phe Thr Val Thr Leu Val Leu Leu Asn Ser Leu Ala Phe Leu Leu
   705                 710                 715                 720
   Met Ala Ile Ile Tyr Thr Lys Leu Tyr Cys Asn Leu Glu Lys Glu Asp
                   725                 730                 735
   Leu Ser Glu Asn Ser Gln Ser Ser Val Ile Lys His Val Ala Trp Leu
               740                 745                 750
   Ile Phe Thr Asn Cys Ile Phe Phe Cys Pro Val Ala Phe Phe Ser Phe
           755                 760                 765
   Ala Pro Leu Ile Thr Ala Ile Ser Ile Ser Pro Glu Ile Met Lys Ser
       770                 775                 780
   Val Thr Leu Ile Phe Phe Pro Leu Pro Ala Cys Leu Asn Pro Val Leu
   785                 790                 795                 800
   Tyr Val Phe Phe Asn Pro Lys Phe Lys Glu Asp Trp Lys Leu Leu Lys
                   805                 810                 815
   Arg Arg Val Thr Arg Lys His Gly Ser Val Ser Val Ser Ile Ser Ser
               820                 825                 830
   Gln Gly Gly Cys Gly Glu Gln Asp Phe Tyr Tyr Asp Cys Gly Met Tyr
           835                 840                 845
   Ser His Leu Gln Gly Asn Leu Thr Val Cys Asp Cys Cys Glu Ser Phe
       850                 855                 860
   Leu Leu Thr Lys Pro Val Ser Cys Lys His Leu Ile Lys Ser His Ser
   865                 870                 875                 880
   Cys Pro Val Leu Thr Ala Ala Ser Cys Gln Arg Pro Glu Ala Tyr Trp
                   885                 890                 895
   Ser Asp Cys Gly Thr Gln Ser Ala His Ser Asp Tyr Ala Asp Glu Glu
               900                 905                 910
   Asp Ser Phe Val Ser Asp Ser Ser Asp Gln Val Gln Ala Cys Gly Arg
           915                 920                 925
   Ala Cys Phe Tyr Gln Ser Arg Gly Phe Pro Leu Val Arg Tyr Ala Tyr
       930                 935                 940
   Asn Leu Gln Arg Val Arg Asp
   945                 950
<210> 3
   <211> 2082
   <212> DNA
   <213> human
<400> 3
   ctacatctcc ataacaatag aatccactcc ctgggaaaga aatgctttga tgggctccac      60
   agcctagaga ctttagattt aaattacaat aaccttgatg aattccccac tgcaattagg     120
   acactctcca acttaaagga actaggattt catagcaaca atatcaggtc gatacctgag     180
   aaagcatttg taggcaaccc ttctcttatt acaatacatt tctatgacaa tcccatccaa     240
   tttgttggga gatctgcttt tcaacattta cctgaactaa gaacactgac tctgaatggt     300
   gcctcacaaa taactgaatt tcctgattta actggaactg caaacctgga gagtctgact     360
   ttaactggag cacagatctc atctcttcct caaaccgtct gcaatcagtt acctaatctc     420
   caagtgctag atctgtctta caacctatta gaagatttac ccagtttttc agtctgccaa     480
   aagcttcaga aaattgacct aagacataat gaaatctacg aaattaaagt tgacactttc     540
   cagcagttgc ttagcctccg atcgctgaat ttggcttgga acaaaattgc tattattcac     600
   cccaatgcat tttccacttt gccatcccta ataaagctgg acctatcgtc caacctcctg     660
   tcgtcttttc ctataactgg gttacatggt ttaactcact taaaattaac aggaaatcat     720
   gccttacaga gctggatatc atctgaaaac tttccagaac tcaaggtnat agaaatgcct     780
   tatgcttacc agtgctgtgc atttggagtg tgtgagaatg cctataagat ttctaatcaa     840
   tggaataaag gtgacaacag cagtatggac gaccttcata agaaagatgc tggaatgttt     900
   caggctcaag atgaacgtga ccttgaagat ttcctgcttg actttgagga agacctgaaa     960
   gcccttcatt cagtgcagtg ttcaccttcc ccaggcccct tcaaaccctg tgaacacctg    1020
   cttgatggct ggctgatcag aattggagtg tggaccatag cagttctggc acttacttgt    1080
   aatgctttgg tgacttcaac agttttcaga tcccctctgt acatttcccc cattaaactg    1140
   ttaattgggg tcatcgcagc agtgaacatg ctcacgggag tctccagtgc cgtgctggct    1200
   ggtgtggatg cgttcacttt tggcagcttt gcacgacatg gtgcctggtg ggagaatggg    1260
   gttggttgcc atgtcattgg ttttttgtcc atttttgctt cagaatcatc tgttttcctg    1320
   cttactctgg cagccctgga gcgtgggttc tctgtgaaat attctgcaaa atttgaaacg    1380
   aaagctccat tttctagcct gaaagtaatc attttgctct gtgccctgct ggccttgacc    1440
   atggccgcag ttcccctgct gggtggcagc aagtatggcg cctcccctct ctgcctgcct    1500
   ttgccttttg  gggagcccag caccatgggc tacatggtcg ctctcatctt gctcaattcc    1560
   ctttgcttcc tcatgatgac cattgcctac accaagctct actgcaattt ggacaaggga    1620
   gacctggaga atatttggga ctgctctatg gtaaaacaca ttgccctgtt gctcttcacc    1680
   aactgcatcc taaactgccc tgtggctttc ttgtccttct cctctttaat aaaccttaca    1740
   tttatcagtc ctgaagtaat taagtttatc cttctggtgg tagtcccact tcctgcatgt    1800
   ctcaatcccc ttctctacat cttgttcaat cctcacttta aggaggatct ggtgagcctg    1860
   agaaagcaaa cctacgtctg gacaagatca aaacacccaa gcttgatgtc aattaactct    1920
   gatgatgtcg aaaaacagtc ctgtgactca actcaagcct tggtaacctt taccagctcc    1980
   agcatcactt atgacctgcc tcccagttcc gtgccatcac cagcttatcc agtgactgag    2040
   agctgccatc tttcctctgt ggcatttgtc ccatgtctct aa                       2082
<210> 4
   <211> 693
   <212> PRT
   <213> human
<400> 4
   Leu His Leu His Asn Asn Arg Ile His Ser Leu Gly Lys Lys Cys Phe
    1               5                  10                  15
   Asp Gly Leu His Ser Leu Glu Thr Leu Asp Leu Asn Tyr Asn Asn Leu
               20                  25                  30
   Asp Glu Phe Pro Thr Ala Ile Arg Thr Leu Ser Asn Leu Lys Glu Leu
           35                  40                  45
   Gly Phe His Ser Asn Asn Ile Arg Ser Ile Pro Glu Lys Ala Phe Val
       50                  55                  60
   Gly Asn Pro Ser Leu Ile Thr Ile His Phe Tyr Asp Asn Pro Ile Gln
   65                  70                  75                  80
   Phe Val Gly Arg Ser Ala Phe Gln His Leu Pro Glu Leu Arg Thr Leu
                   85                  90                  95
   Thr Leu Asn Gly Ala Ser Gln Ile Thr Glu Phe Pro Asp Leu Thr Gly
               100                 105                 110
   Thr Ala Asn Leu Glu Ser Leu Thr Leu Thr Gly Ala Gln Ile Ser Ser
           115                 120                 125
   Leu Pro Gln Thr Val Cys Asn Gln Leu Pro Asn Leu Gln Val Leu Asp
       130                 135                 140
   Leu Ser Tyr Asn Leu Leu Glu Asp Leu Pro Ser Phe Ser Val Cys Gln
   145                 150                 155                 160
   Lys Leu Gln Lys Ile Asp Leu Arg His Asn Glu Ile Tyr Glu Ile Lys
                   165                 170                 175
   Val Asp Thr Phe Gln Gln Leu Leu Ser Leu Arg Ser Leu Asn Leu Ala
               180                 185                 190
   Trp Asn Lys Ile Ala Ile Ile His Pro Asn Ala Phe Ser Thr Leu Pro
           195                 200                 205
   Ser Leu Ile Lys Leu Asp Leu Ser Ser Asn Leu Leu Ser Ser Phe Pro
       210                 215                 220
   Ile Thr Gly Leu His Gly Leu Thr His Leu Lys Leu Thr Gly Asn His
   225                 230                 235                 240
   Ala Leu Gln Ser Trp Ile Ser Ser Glu Asn Phe Pro Glu Leu Lys Val
                   245                 250                 255
   Ile Glu Met Pro Tyr Ala Tyr Gln Cys Cys Ala Phe Gly Val Cys Glu
               260                 265                 270
   Asn Ala Tyr Lys Ile Ser Asn Gln Trp Asn Lys Gly Asp Asn Ser Ser
           275                 280                 285
   Met Asp Asp Leu His Lys Lys Asp Ala Gly Met Phe Gln Ala Gln Asp
       290                 295                 300
   Glu Arg Asp Leu Glu Asp Phe Leu Leu Asp Phe Glu Glu Asp Leu Lys
   305                 310                 315                 320
   Ala Leu His Ser Val Gln Cys Ser Pro Ser Pro Gly Pro Phe Lys Pro
                   325                 330                 335
   Cys Glu His Leu Leu Asp Gly Trp Leu Ile Arg Ile Gly Val Trp Thr
               340                 345                 350
   Ile Ala Val Leu Ala Leu Thr Cys Asn Ala Leu Val Thr Ser Thr Val
           355                 360                 365
   Phe Arg Ser Pro Leu Tyr Ile Ser Pro Ile Lys Leu Leu Ile Gly Val
       370                 375                 380
   Ile Ala Ala Val Asn Met Leu Thr Gly Val Ser Ser Ala Val Leu Ala
   385                 390                 395                 400
   Gly Val Asp Ala Phe Thr Phe Gly Ser Phe Ala Arg His Gly Ala Trp
                   405                 410                 415
   Trp Glu Asn Gly Val Gly Cys His Val Ile Gly Phe Leu Ser Ile Phe
               420                 425                 430
   Ala Ser Glu Ser Ser Val Phe Leu Leu Thr Leu Ala Ala Leu Glu Arg
           435                 440                 445
   Gly Phe Ser Val Lys Tyr Ser Ala Lys Phe Glu Thr Lys Ala Pro Phe
       450                 455                 460
   Ser Ser Leu Lys Val Ile Ile Leu Leu Cys Ala Leu Leu Ala Leu Thr
   465                 470                 475                 480
   Met Ala Ala Val Pro Leu Leu Gly Gly Ser Lys Tyr Gly Ala Ser Pro
                   485                 490                 495
   Leu Cys Leu Pro Leu Pro Phe Gly Glu Pro Ser Thr Met Gly Tyr Met
               500                 505                 510
   Val Ala Leu Ile Leu Leu Asn Ser Leu Cys Phe Leu Met Met Thr Ile
           515                 520                 525
   Ala Tyr Thr Lys Leu Tyr Cys Asn Leu Asp Lys Gly Asp Leu Glu Asn
       530                 535                 540
   Ile Trp Asp Cys Ser Met Val Lys His Ile Ala Leu Leu Leu Phe Thr
   545                 550                 555                 560
   Asn Cys Ile Leu Asn Cys Pro Val Ala Phe Leu Ser Phe Ser Ser Leu
                   565                 570                 575
   Ile Asn Leu Thr Phe Ile Ser Pro Glu Val Ile Lys Phe Ile Leu Leu
               580                 585                 590
   Val Val Val Pro Leu Pro Ala Cys Leu Asn Pro Leu Leu Tyr Ile Leu
           595                 600                 605
   Phe Asn Pro His Phe Lys Glu Asp Leu Val Ser Leu Arg Lys Gln Thr
       610                 615                 620
   Tyr Val Trp Thr Arg Ser Lys His Pro Ser Leu Met Ser Ile Asn Ser
   625                 630                 635                 640
   Asp Asp Val Glu Lys Gln Ser Cys Asp Ser Thr Gln Ala Leu Val Thr
                   645                 650                 655
   Phe Thr Ser Ser Ser Ile Thr Tyr Asp Leu Pro Pro Ser Ser Val Pro
               660                 665                 670
   Ser Pro Ala Tyr Pro Val Thr Glu Ser Cys His Leu Ser Ser Val Ala
           675                 680                 685
   Phe Val Pro Cys Leu
       690
<210> 5
   <211> 2467
   <212> DNA
   <213> human
<400> 5
   gaaaggagga aagaaaaaaa gaggaatgga aagagacaga gaaaggaaat gggagtggaa      60
   ggagggagga ctgctttgta actgctaaga ttgcagacag aaatagcaca caaccactgt     120
   gagctgtatg cgattcagaa accaagacca aattttgctc actttcatta atcagttgct     180
   cagatagaag gaaatgacat ctggttctgt cttcttctac atcttaattt ttggaaaata     240
   tttttctcat gggggtggac aggatgtcaa gtgctccctt ggctatttcc cctgtgggaa     300
   catcacaaag tgcttgcctc agctcctgca ctgtaacggt gtggacgact gcgggaatca     360
   ggccgatgag gacaactgtg gagacaacaa tggatggtcc atgcaatttg acaaatattt     420
   tgccagttac tacaaaatga cttcccaata tccttttgag gcagaaacac ctgaatgttt    480
   ggtcggttct gtgccagtgc aatgtctttg ccaaggtctg gagcttgact gtgatgaaac     540
   caatttacga gctgttccat cggtttcttc aaatgtgact gcaatgtcac ttcagtggaa     600
   cttaataaga aagcttcctc ctgattgctt caagaattat catgatcttc agaagctgta     660
   cctgcaaaac aataagatta catccatctc catctatgct ttcagaggac tgaatagcct     720
   tactaaactg tatctcagtc ataacagaat aaccttcctg aagccgggtg tttttgaaga     780
   tcttcacaga ctagaatggc tgataattga agataatcac ctcagtcgaa tttccccacc     840
   aacattttat ggactaaatt ctcttattct cttagtcctg atgaataacg tcctcacccg     900
   tttacctgat aaacctctct gtcaacacat gccaagacta cattggctgg accttgaagg     960
   caaccatatc cataatttaa gaaatttgac ttttatttcc tgcagtaatt taactgtttt    1020
   agtgatgagg aaaaacaaaa ttaatcactt aaatgaaaat acttttgcac ctctccagaa    1080
   actggatgaa ttggatttag gaagtaataa gattgaaaat cttccaccgc ttatattcaa    1140
   ggacctgaag gagctgtcac aattgaatct ttcctataat ccaatccaga aaattcaagc    1200
   aaaccaattt gattatcttg tcaaactcaa gtctctcagc ctagaaggga ttgaaatttc    1260
   aaatatccaa caaaggatgt ttagacctct tatgaatctc tctcacatat attttaagaa    1320
   attccagtac tgtgggtatg caccacatgt tcgcagctgt aaaccaaaca ctgatggaat    1380
   ttcatctcta gagaatctct tggcaagcat tattcagaga gtatttgtct gggttgtatc    1440
   tgcagttacc tgctttggaa acatttttgt catttgcatg cgaccttata tcaggtctga    1500
   gaacaagctg tatgccatgt caatcatttc tctctgctgt gccgactgct taatgggaat    1560
   atatttattc gtgatcggag gctttgacct aaagtttcgt ggagaataca ataagcatgc    1620
   gcagctgtgg atggagagta ctcattgtca gcttgtagga tctttggcca ttctgtccac    1680
   agaagtatca gttttactgt taacatttct gacattggaa aaatacatct gcattgtcta    1740
   tccttttaga tgtgtgagac ctggaaaatg cagaacaatt acagttctga ttctcatttg    1800
   gattactggt tttatagtgg ctttcattcc attgagcaat aaggaatttt tcaaaaacta    1860
   ctatggcacc aatggagtat gcttccctct tcattcagaa gatacagaaa gtattggagc    1920
   ccagatttat tcagtggcaa tttttcttgg tattaatttg gccgcattta tcatcatagt    1980
   tttttcctat ggaagcatgt tttatagtgt tcatcaaagt gccataacag caactgaaat    2040
   acggaatcaa gttaaaaaag agatgatcct tgccaaacgt tttttcttta tagtatttac    2100
   tgatgcatta tgctggatac ccatttttgt agtgaaattt ctttcactgc ttcaggtaga    2160
   aataccaggt accataacct cttgggtagt gatttttatt ctgcccatta acagtgcttt    2220
   gaacccaatt ctctatactc tgaccacaag accatttaaa gaaatgattc atcggttttg    2280
   gtataactac agacaaagaa aatctatgga cagcaaaggt cagaaaacat atgctccatc    2340
   attcatctgg gtggaaatgt ggccactgca ggagatgcca cctgagttaa tgaagccgga    2400
   ccttttcaca tacccctgtg aaatgtcact gatttctcaa tcaacgagac tcaattccta    2460
   ttcatga                                                               2467
<210> 6
   <211> 757
   <212> PRT
   <213> human
<400> 6
   Met Thr Ser Gly Ser Val Phe Phe Tyr Ile Leu Ile Phe Gly Lys Tyr
    1               5                  10                  15
   Phe Ser His Gly Gly Gly Gln Asp Val Lys Cys Ser Leu Gly Tyr Phe
               20                  25                  30
   Pro Cys Gly Asn Ile Thr Lys Cys Leu Pro Gln Leu Leu His Cys Asn
           35                  40                  45
   Gly Val Asp Asp Cys Gly Asn Gln Ala Asp Glu Asp Asn Cys Gly Asp
       50                  55                  60
   Asn Asn Gly Trp Ser Met Gln Phe Asp Lys Tyr Phe Ala Ser Tyr Tyr
   65                  70                  75                  80
   Lys Met Thr Ser Gln Tyr Pro Phe Glu Ala Glu Thr Pro Glu Cys Leu
                   85                  90                  95
   Val Gly Ser Val Pro Val Gln Cys Leu Cys Gln Gly Leu Glu Leu Asp
               100                 105                 110
   Cys Asp Glu Thr Asn Leu Arg Ala Val Pro Ser Val Ser Ser Asn Val
           115                 120                 125
   Thr Ala Met Ser Leu Gln Trp Asn Leu Ile Arg Lys Leu Pro Pro Asp
       130                 135                 140
   Cys Phe Lys Asn Tyr His Asp Leu Gln Lys Leu Tyr Leu Gln Asn Asn
   145                 150                 155                 160
   Lys Ile Thr Ser Ile Ser Ile Tyr Ala Phe Arg Gly Leu Asn Ser Leu
                   165                 170                 175
   Thr Lys Leu Tyr Leu Ser His Asn Arg Ile Thr Phe Leu Lys Pro Gly
               180                 185                 190
   Val Phe Glu Asp Leu His Arg Leu Glu Trp Leu Ile Ile Glu Asp Asn
           195                 200                 205
   His Leu Ser Arg Ile Ser Pro Pro Thr Phe Tyr Gly Leu Asn Ser Leu
       210                 215                 220
   Ile Leu Leu Val Leu Met Asn Asn Val Leu Thr Arg Leu Pro Asp Lys
   225                 230                 235                 240
   Pro Leu Cys Gln His Met Pro Arg Leu His Trp Leu Asp Leu Glu Gly
                   245                 250                 255
   Asn His Ile His Asn Leu Arg Asn Leu Thr Phe Ile Ser Cys Ser Asn
               260                 265                 270
   Leu Thr Val Leu Val Met Arg Lys Asn Lys Ile Asn His Leu Asn Glu
           275                 280                 285
   Asn Thr Phe Ala Pro Leu Gln Lys Leu Asp Glu Leu Asp Leu Gly Ser
       290                 295                 300
   Asn Lys Ile Glu Asn Leu Pro Pro Leu Ile Phe Lys Asp Leu Lys Glu
   305                 310                 315                 320
   Leu Ser Gln Leu Asn Leu Ser Tyr Asn Pro Ile Gln Lys Ile Gln Ala
                   325                 330                 335
   Asn Gln Phe Asp Tyr Leu Val Lys Leu Lys Ser Leu Ser Leu Glu Gly
               340                 345                 350
   Ile Glu Ile Ser Asn Ile Gln Gln Arg Met Phe Arg Pro Leu Met Asn
           355                 360                 365
   Leu Ser His Ile Tyr Phe Lys Lys Phe Gln Tyr Cys Gly Tyr Ala Pro
       370                 375                 380
   His Val Arg Ser Cys Lys Pro Asn Thr Asp Gly Ile Ser Ser Leu Glu
   385                 390                 395                 400
   Asn Leu Leu Ala Ser Ile Ile Gln Arg Val Phe Val Trp Val Val Ser
                   405                 410                 415
   Ala Val Thr Cys Phe Gly Asn Ile Phe Val Ile Cys Met Arg Pro Tyr
               420                 425                 430
   Ile Arg Ser Glu Asn Lys Leu Tyr Ala Met Ser Ile Ile Ser Leu Cys
           435                 440                 445
   Cys Ala Asp Cys Leu Met Gly Ile Tyr Leu Phe Val Ile Gly Gly Phe
       450                 455                 460
   Asp Leu Lys Phe Arg Gly Glu Tyr Asn Lys His Ala Gln Leu Trp Met
   465                 470                 475                 480
   Glu Ser Thr His Cys Gln Leu Val Gly Ser Leu Ala Ile Leu Ser Thr
                   485                 490                 495
   Glu Val Ser Val Leu Leu Leu Thr Phe Leu Thr Leu Glu Lys Tyr Ile
               500                 505                 510
   Cys Ile Val Tyr Pro Phe Arg Cys Val Arg Pro Gly Lys Cys Arg Thr
           515                 520                 525
   Ile Thr Val Leu Ile Leu Ile Trp Ile Thr Gly Phe Ile Val Ala Phe
       530                 535                 540
   Ile Pro Leu Ser Asn Lys Glu Phe Phe Lys Asn Tyr Tyr Gly Thr Asn
   545                 550                 555                 560
   Gly Val Cys Phe Pro Leu His Ser Glu Asp Thr Glu Ser Ile Gly Ala
                   565                 570                 575
   Gln Ile Tyr Ser Val Ala Ile Phe Leu Gly Ile Asn Leu Ala Ala Phe
               580                 585                 590
   Ile Ile Ile Val Phe Ser Tyr Gly Ser Met Phe Tyr Ser Val His Gln
           595                 600                 605
   Ser Ala Ile Thr Ala Thr Glu Ile Arg Asn Gln Val Lys Lys Glu Met
       610                 615                 620
   Ile Leu Ala Lys Arg Phe Phe Phe Ile Val Phe Thr Asp Ala Leu Cys
   625                 630                 635                     640
   Trp Ile Pro Ile Phe Val Val Lys Phe Leu Ser Leu Leu Gln Val Glu
                   645                 650                 655
   Ile Pro Gly Thr Ile Thr Ser Trp Val Val Ile Phe Ile Leu Pro Ile
               660                 665                 670
   Asn Ser Ala Leu Asn Pro Ile Leu Tyr Thr Leu Thr Thr Arg Pro Phe
           675                 680                 685
   Lys Glu Met Ile His Arg Phe Trp Tyr Asn Tyr Arg Gln Arg Lys Ser
       690                 695                 700
   Met Asp Ser Lys Gly Gln Lys Thr Tyr Ala Pro Ser Phe Ile Trp Val
   705                 710                 715                     720
   Glu Met Trp Pro Leu Gln Glu Met Pro Pro Glu Leu Met Lys Pro Asp
                   725                 730                 735
   Leu Phe Thr Tyr Pro Cys Glu Met Ser Leu Ile Ser Gln Ser Thr Arg
               740                 745                 750
   Leu Asn Ser Tyr Ser
           755
<210> 7
   <211> 3584
   <212> DNA
   <213> human
<400> 7
   ctgctttgta actgctaaga ttgcagacag aaatagcaca caaccactgt gagctgtatg      60
   cgattcagaa accaagacca aattttgctc actttcatta atcagttgct cagatagaag     120
   gaaatgacat ctggttctgt cttcttctac atcttaattt ttggaaaata tttttctcat     180
   gggggtggac aggatgtcaa gtgctccctt ggctatttcc cctgtgggaa catcacaaag     240
   tgcttgcctc agctcctgca ctgtaacggt gtggacgact gcgggaatca ggccgatgag     300
   gacaactgtg tggtggtttt gtgccagtgc atgtctttgc caggtctgga gcttgactgg     360
   atgaaaccat ttacgagtgt tccatcggtt tcttcaaatg tgactgcaat gtcacttcag     420
   tggaacttaa taagaaagct tcctcctgat tgcttcaaga attatcatga tcttcagaag     480
   ctggacctgc aaaacaataa gattacatcc atctccatct atgctttcag aggactgaat     540
   agccttacta aactgtatct cagtcataac agaataacct tcctgaagcc gggtgttttt     600
   gaagatcttc acagactaga atggctgata attgaagata atcacctcag tcgaatttcc     660
   ccaccaacat tttatggact aaattctctt attctcttag tcctgatgaa taacgtcctc     720
   acccgtttac ctgataaacc tctctgtcaa cacatgccaa gactacattg gctggacctt     780
   gaaggcaacc atatccataa tttaagaaat ttgactttta tttcctgcag taatttaact     840
   gttttagtga tgaggaaaaa caaaattaat cacttaaatg aaaatacttt tgcacctctc     900
   cagaaactgg atgaattgga tttaggaagt aataagattg aaaatcttcc accgcttata     960
   ttcaaggacc tgaaggagct gtcacaattg aatctttcct ataatccaat ccagaaaatt    1020
   caagcaaacc aatttgatta tcttgtcaaa ctcaagtctc tcagcctaga agggattgaa    1080
   atttcaaata tccaacaaag gatgtttaga cctcttatga atctctctca catatatttt    1140
   aagaaattcc agtactgtgg gtatgcacca catgttcgca gctgtaaacc aaacactgat    1200
   ggaatttcat ctctagagaa tctcttggca agcattattc agagagtatt tgtctgggtt    1260
   gtatctgcag ttacctgctt tggaaacatt tttgtcattt gcatgcgacc ttatatcagg    1320
   tctgagaaca agctgtatgc catgtcaatc atttctctct gctgtgccga ctgcttaatg    1380
   ggaatatatt tattcgtgat cggaggcttt gacctaaagt ttcgtggaga atacaataag    1440
   catgcgcagc tgtggatgga gagtactcat tgtcagcttg taggatcttt ggccattctg    1500
   tccacagaag tatcagtttt actgttaaca tttctgacat tggaaaaata catctgcatt    1560
   gtctatcctt ttagatgtgt gagacctgga aaatgcagaa caattacagt tctgattctc    1620
   atttggatta ctggttttat agtggctttc attccattga gcaataagga atttttcaaa    1680
   aactactatg gcaccaatgg agtatgcttc cctcttcatt cagaagatac agaaagtatt    1740
   ggagcccaga tttattcagt ggcaattttt cttggtatta atttggccgc atttatcatc    1800
   atagtttttt cctatggaag catgttttat agtgttcatc aaagtgccat aacagcaact    1860
   gaaatacgga atcaagttaa aaaagagatg atccttgcca aacgtttttt ctttatagta    1920
   tttactgatg cattatgctg gatacccatt tttgtagtga aatttctttc actgcttcag    1980
   gtagaaatac caggtaccat aacctcttgg gtagtgattt ttattctgcc cattaacagt    2040
   gctttgaacc caattctcta tactctgacc acaagaccat ttaaagaaat gattcatcgg    2100
   ttttggtata actacagaca aagaaaatct atggacagca aaggtcagaa aacatatgct    2160
   ccatcattca tctgggtgga aatgtggcca ctgcaggaga tgccacctga gttaatgaag    2220
   ccggaccttt tcacataccc ctgtgaaatg tcactgattt ctcaatcaac gagactcaat    2280
   tcctattcat gactgactct gaaattcatt tcttcgcaga gaatactgtg ggggtgcttc    2340
   atgagggatt tactggtatg aaaatgaata ccacaaaatt aatttataat aatagctaag    2400
   ataaatattt tacaaggaca tgaggaaaaa taaaaatgac taatgctctt acaaagggaa    2460
   gtaattatat caataatgta tatatattag tagacatttt gcataagaaa ttaagagaaa    2520
   tctacttcag taacattcat tcatttttct aacatgcatt tattgagtac ccactactat    2580
   gtgcatagca ttgcaatata gtcctggaag tagacagtgc agaacctttc aatctgtaga    2640
   tagtgtttaa tgacaaaaga ctatacaaag tccatctgca gttcctagtt taaagtagag    2700
   ctttacctgt catgtgcatc agcaagaatc ataggcactt ttaaataaag gtttaaagtt    2760
   ttggaatact cagtgtattt gcatcataga aaatgtctga ctgtttgcaa aataatattc    2820
   tgttttaaga atccatctta cctctcttta agtttccata cacttgagag ccaacacaac    2880
   atatttatta ctaaaaagat gctttgctag aaactcaaaa acagcacttc ttttggcact    2940
   tcctgcccag ttttctcttt gctttaaatg aacatcatca tatggaattg gaataggaga    3000
   gtatgagtac ggcagagaag tggatcagaa aaactagaat gaggataaac atttacatta    3060
   gtggaaactc ctgaaataaa tccttgtatt gtcagttaac tgattttcaa caaggatgcc    3120
   aagacaaaaa ggcttttcaa caaaccgtgc tgttttaaga acagacctaa gtggtttaat    3180
   tcacccactt tagatgggtg aatgttatgg tgtgtgaaat atctcagtaa agcagttaaa    3240
   aggaaaaaga gctggaatgc actgattcag gaacttaatt tcaggaagga aaggtctgta    3300
   tgtacacatt tcactttaag cagaaaatct ttcttcaaga aatgacttta ctttctcttt    3360
   gcactgccag cacgtgagat actaactttt taactagttg ttcttctcta gtctctacgt    3420
   tattagnatt ttttgctttc ataatgtgaa acctttaagc aggagaagaa aatgttttca    3480
   gatagtttca aatacnccaa aaatgtttgc aacacaaaaa tactggaatc naaccataat    3540
   gcccttattg aatatatagt tgtatagntt tgttctgaaa accc                     3584
<210> 8
   <211> 722
   <212> PRT
   <213> human
<400> 8
   Met Thr Ser Gly Ser Val Phe Phe Tyr Ile Leu Ile Phe Gly Lys Tyr
    1               5                  10                  15
   Phe Ser His Gly Gly Gly Gln Asp Val Lys Cys Ser Leu Gly Tyr Phe
               20                  25                  30
   Pro Cys Gly Asn Ile Thr Lys Cys Leu Pro Gln Leu Leu His Cys Asn
           35                  40                  45
   Gly Val Asp Asp Cys Gly Asn Gln Ala Asp Glu Asp Asn Cys Val Val
       50                  55                  60
   Val Leu Cys Gln Cys Met Ser Leu Pro Gly Leu Glu Leu Asp Trp Met
   65                  70                  75                  80
   Lys Pro Phe Thr Ser Val Pro Ser Val Ser Ser Asn Val Thr Ala Met
                   85                  90                  95
   Ser Leu Gln Trp Asn Leu Ile Arg Lys Leu Pro Pro Asp Cys Phe Lys
               100                 105                 110
   Asn Tyr His Asp Leu Gln Lys Leu Asp Leu Gln Asn Asn Lys Ile Thr
           115                 120                 125
   Ser Ile Ser Ile Tyr Ala Phe Arg Gly Leu Asn Ser Leu Thr Lys Leu
       130                 135                 140
   Tyr Leu Ser His Asn Arg Ile Thr Phe Leu Lys Pro Gly Val Phe Glu
   145                 150                 155                 160
   Asp Leu His Arg Leu Glu Trp Leu Ile Ile Glu Asp Asn His Leu Ser
                   165                 170                 175
   Arg Ile Ser Pro Pro Thr Phe Tyr Gly Leu Asn Ser Leu Ile Leu Leu
               180                 185                 190
   Val Leu Met Asn Asn Val Leu Thr Arg Leu Pro Asp Lys Pro Leu Cys
   195 200 205
   Gln His Met Pro Arg Leu His Trp Leu Asp Leu Glu Gly Asn His Ile
   210 215 220
   His Asn Leu Arg Asn Leu Thr Phe Ile Ser Cys Ser Asn Leu Thr Val
   225 230 235 240
   Leu Val Met Arg Lys Asn Lys Ile Asn His Leu Asn Glu Asn Thr Phe
   245 250 255
   Ala Pro Leu Gln Lys Leu Asp Glu Leu Asp Leu Gly Ser Asn Lys Ile
   260 265 270
   Glu Asn Leu Pro Pro Leu Ile Phe Lys Asp Leu Lys Glu Leu Ser Gln
   275 280 285
   Leu Asn Leu Ser Tyr Asn Pro Ile Gln Lys Ile Gln Ala Asn Gln Phe
   290 295 300
   Asp Tyr Leu Val Lys Leu Lys Ser Leu Ser Leu Glu Gly Ile Glu Ile
   305 310 315 320
   Ser Asn Ile Gln Gln Arg Met Phe Arg Pro Leu Met Asn Leu Ser His
   325 330 335
   Ile Tyr Phe Lys Lys Phe Gln Tyr Cys Gly Tyr Ala Pro His Val Arg
   340 345 350
   Ser Cys Lys Pro Asn Thr Asp Gly Ile Ser Ser Leu Glu Asn Leu Leu
   355 360 365
   Ala Ser Ile Ile Gln Arg Val Phe Val Trp Val Val Ser Ala Val Thr
   370 375 380
   Cys Phe Gly Asn Ile Phe Val Ile Cys Met Arg Pro Tyr Ile Arg Ser
   385 390 395 400
   Glu Asn Lys Leu Tyr Ala Met Ser Ile Ile Ser Leu Cys Cys Ala Asp
   405 410 415
   Cys Leu Met Gly Ile Tyr Leu Phe Val Ile Gly Gly Phe Asp Leu Lys
   420 425 430
   Phe Arg Gly Glu Tyr Asn Lys His Ala Gln Leu Trp Met Glu Ser Thr
   435 440 445
   His Cys Gln Leu Val Gly Ser Leu Ala Ile Leu Ser Thr Glu Val Ser
   450 455 460
   Val Leu Leu Leu Thr Phe Leu Thr Leu Glu Lys Tyr Ile Cys Ile Val
   465 470 475 480
   Tyr Pro Phe Arg Cys Val Arg Pro Gly Lys Cys Arg Thr Ile Thr Val
   485 490 495
   Leu Ile Leu Ile Trp Ile Thr Gly Phe Ile Val Ala Phe Ile Pro Leu
   500 505 510
   Ser Asn Lys Glu Phe Phe Lys Asn Tyr Tyr Gly Thr Asn Gly Val Cys
   515 520 525
   Phe Pro Leu His Ser Glu Asp Thr Glu Ser Ile Gly Ala Gln Ile Tyr
   530 535 540
   Ser Val Ala Ile Phe Leu Gly Ile Asn Leu Ala Ala Phe Ile Ile Ile
   545 550 555 560
   Val Phe Ser Tyr Gly Ser Met Phe Tyr Ser Val His Gln Ser Ala Ile
   565 570 575
   Thr Ala Thr Glu Ile Arg Asn Gln Val Lys Lys Glu Met Ile Leu Ala
   580 585 590
   Lys Arg Phe Phe Phe Ile Val Phe Thr Asp Ala Leu Cys Trp Ile Pro
   595 600 605
   Ile Phe Val Val Lys Phe Leu Ser Leu Leu Gln Val Glu Ile Pro Gly
   610 615 620
   Thr Ile Thr Ser Trp Val Val Ile Phe Ile Leu Pro Ile Asn Ser Ala
   625 630 635 640
   Leu Asn Pro Ile Leu Tyr Thr Leu Thr Thr Arg Pro Phe Lys Glu Met
   645 650 655
   Ile His Arg Phe Trp Tyr Asn Tyr Arg Gln Arg Lys Ser Met Asp Ser
               660                 665                 670
   Lys Gly Gln Lys Thr Tyr Ala Pro Ser Phe Ile Trp Val Glu Met Trp
           675                 680                 685
   Pro Leu Gln Glu Met Pro Pro Glu Leu Met Lys Pro Asp Leu Phe Thr
       690                 695                 700
   Tyr Pro Cys Glu Met Ser Leu Ile Ser Gln Ser Thr Arg Leu Asn Ser
   705                 710                 715                 720
   Tyr Ser